# EUROPEAN PATENT SPECIFICATION

(11) **EP 3 572 434 B1**
(45) Date of publication and mention of the grant of the patent: **17.08.2022**
(21) Application number: 18174279.2
(22) Date of filing: 25.05.2018
(51) Int. Cl.: C08B 11/187, C08B 11/20, C08B 15/00, C08J 3/075, C08L 1/02, A61K 47/69, A61K 47/61

(54) **NANOFIBRILLAR CELLULOSE HYDROGEL**
NANOFIBRILLÄRES CELLULOSEHYDROGEL
HYDROGEL DE CELLULOSE NANOFIBRILLAIRE

(43) Date of publication of application: 27.11.2019
(73) Proprietor: UPM-Kymmene Corporation, 00100 Helsinki (FI)
(72) Inventor: Nuopponen, Markus, 00220 Helsinki (FI); Paasonen, Lauri, 04440 Järvenpää (FI); Satomaa, Tero, 00700 Helsinki (FI); Aitio, Olli, 00330 Helsinki (FI); Helin, Jari, 05200 Rajamäki (FI)
(74) Representative: Papula Oy

(56) References cited:
- WO-A1-2015/189478
- ANDREAS KOSCHELLA ET AL: "A click-chemistry approach to cellulose-based hydrogels", CARBOHYDRATE POLYMERS, vol. 86, no. 1, 12 April 2011 (2011-04-12) , pages 154-161, XP028378205, APPLIED SCIENCE PUBLISHERS, LTD. BARKING, GB ISSN: 0144-8617, DOI: 10.1016/J.CARBPOL.2011.04.031 [retrieved on 2011-04-21]
- ZHENG, JUNG, SCHMIDT, LODGE, REINEKE: "2-hydroxyethylcellulose and amphiphilic block copolymer conjugates form mechanically tunable and nonswellable hydrogel", ACS MACRO LETTERS, vol. 6, 30 January 2017 (2017-01-30), pages 145-149, XP002786706, DOI: 10.1021/acsmacrolett.6b00954

## Description

### TECHNICAL FIELD

The present disclosure relates to a nanofibrillar cellulose hydrogel.

### BACKGROUND

Nanofibrillar cellulose hydrogel is used in 3D cell culture, as the hydrogel provides a three-dimensional matrix in which cells can grow and with which they can interact. The nanofibrillar cellulose hydrogel is a relatively defined cell culture substrate and as such does not usually contain growth factors or other components intended to affect the growth, differentiation or adherence of cells thereto, apart from the nanofibrillar cellulose itself.

The nanofibrillar cellulose hydrogel is typically delivered as a hydrogel dispersion with a viscosity and other rheological properties suitable for cell culture.

### SUMMARY

A nanofibrillar cellulose hydrogel is disclosed. The nanofibrillar cellulose hydrogel comprises azido-modified nanofibrillar cellulose. The azido-modified nanofibrillar cellulose has a substituent represented by the formula -O-(CH₂)ₙ-S(O)ₘ-L₁-N₃, wherein n is in the range of 1 to 10; m is 0 or 1; and L₁ is a linker. The substituent is attached to a carbon of one or more glucosyl units of the azido-modified nanofibrillar cellulose, thus forming an ether bond to the carbon.

### BRIEF DESCRIPTION OF THE DRAWINGS

The accompanying drawings, which are included to provide a further understanding of the embodiments and constitute a part of this specification, illustrate various embodiments. In the drawings:
Fig. 1: A schematic outline of the generation of azido-modified and ligand-modified nanofibrillar cellulose;
Fig. 2: MALDI-TOF mass spectrometry of azido-modified GrowDex^{®};
Fig. 3: Modified GrowDex^{®} is functional after autoclaving;
Fig. 4: ¹H-NMR spectroscopy of allylated GrowDex^{®};
Figs. 5A-5D: iPS cells in the different 3D hydrogels after 9 days of culture;
Fig. 6: Cell counts of iPS cells in different 3D hydrogels;
Fig. 7: Cell viability in different 3D hydrogels;
Fig. 8: Cell proliferation assay;
Figs. 9A and 9B: Anti-Tra-1-60 staining in spheroids grown in modified GrowDex^{®};
Figs. 10A and 10B: Anti-SSEA-4 staining in spheroids grown in ECA-GrowDex^{®};
Figs. 11A-11C: Alexa488-phalloidin staining in a "large" spheroid grown in ECA-GrowDex^{®};
Figs. 12A-12C: Alexa488-phalloidin staining in a spheroid grown in glycan1-GrowDex^{®};
Figs. 13A-13C: Alexa488-phalloidin staining in a spheroid grown in unmodified GrowDex^{®};
Figs. 14A-14C: Alexa488-phalloidin staining in a spheroid grown in unmodified GrowDex^{®};and
Fig. 15: The visco-elastic properties of 0.5 % nanocellulose dispersions of unmodified sample (solid line) and modified sample (dotted line) by a stress-sweep measurement.

### DETAILED DESCRIPTION

A nanofibrillar cellulose hydrogel comprising azido-modified nanofibrillar cellulose is disclosed. The azido-modified nanofibrillar cellulose has azido (-N₃) groups covalently bound thereto.

It has now been found that it is possible to use the nanofibrillar cellulose hydrogel comprising the azido-modified nanofibrillar cellulose to covalently link, i.e. conjugate, ligands to the nanofibrillar cellulose of the hydrogel. For example, ligands such as growth factors or other biomolecules can be linked to the hydrogel. Suitable ligands may include e.g. proteins, peptides, glycans or nanofibrillar cellulose (cross-linked nanofibrillar cellulose). Such ligands may improve or support growth, differentiation and/or attachment of cells and/or tissues grown in contact with the hydrogel. The nanofibrillar cellulose hydrogel may be suitable for the culture of cells and/or tissues, for example pluripotent stem (PS) cells, such as induced pluripotent stem cells (iPS cells).

The nanofibrillar cellulose hydrogel can be provided in a form that allows linking one or more ligands of choice and/or one or more ligands in an amount of choice. The linking may be done by an end user shortly prior to use of the hydrogel.

The rheological properties and/or gelling properties of the nanofibrillar cellulose hydrogel are not necessarily significantly affected by the modification(s). The azide modification according to one or more embodiments described in this specification does not seem to cause significant unwanted cross-linking of nanofibrillar cellulose, which might affect rheological or gelling properties adversely.

The reactions for preparing the azido-modified nanofibrillar cellulose hydrogel and for linking the ligand thereto may be performed in an aqueous solution, so the properties of the hydrogel may not be significantly affected by the reaction conditions used when preparing the hydrogel. The reactions do not require e.g. harsh solvents. Further, the chemistry used does not introduce potentially toxic components to the hydrogel. Therefore procedures for purifying the hydrogel after the reactions can be relatively light or may even be obviated.

The modification of the nanofibrillar cellulose does not significantly interfere with enzymatic degradation of the nanofibrillar cellulose hydrogel, for example using one or more cellulases and/or hemicellulases. The ligands may also be distributed relatively uniformly within the hydrogel as compared e.g. to simple mixing of the ligands with a nanofibrillar cellulose hydrogel so that the ligands are in solution and not bound covalently to the nanofibrillar cellulose.

The nanofibrillar cellulose may be prepared from cellulose raw material of plant origin. The raw material may be based on any plant material that contains cellulose. The raw material may also be derived from certain bacterial fermentation processes. In an embodiment the plant material is wood. Wood may be from a softwood tree, such as spruce, pine, fir, larch, douglas-fir or hemlock, or from a hardwood tree, such as birch, aspen, poplar, alder, eucalyptus, oak, beech or acacia, or from a mixture of softwoods and hardwoods. In an embodiment, the nanofibrillar cellulose is obtained from wood pulp. In an embodiment, the nanofibrillar cellulose is obtained from hardwood pulp. In an example, the hardwood is birch. In an embodiment, the nanofibrillar cellulose is obtained from softwood pulp.

The nanofibrillar cellulose may be made of plant material. In an example, the fibrils are obtained from non-parenchymal plant material. In such a case, the fibrils may be obtained from secondary cell walls. One abundant source of such cellulose fibrils is wood fibres. The smallest cellulosic entities of cellulose pulp of plant origin, such as wood, include cellulose molecules, elementary fibrils, and microfibrils. Microfibril units are bundles of elementary fibrils caused by physically conditioned coalescence as a mechanism of reducing the free energy of the surfaces.

The nanofibrillar cellulose is manufactured by homogenizing wood-derived fibrous raw material, which may be chemical pulp. Cellulose fibers may be disintegrated to produce fibrils which have a diameter in the nanometer range, which diameter may be up to 200 nm, or up to 50 nm, for example in the range of 1-200 nm or 1-100 nm, and gives a dispersion of fibrils in water. The fibrils may be reduced to a size in which the diameter of most of the fibrils is in the range of 2-20 nm. The fibrils originating from secondary cell walls may be essentially crystalline, with a degree of crystallinity of at least 55 %. Such fibrils may have different properties than fibrils originated from primary cell walls; for example, the dewatering of fibrils originating from secondary cell walls may be more challenging.

In the context of this specification, the term "nanofibrillar cellulose" may refer to cellulose fibrils or fibril bundles separated from cellulose-based fiber raw material. These fibrils are characterized by a high aspect ratio (length/diameter): their length may exceed 1 µm, whereas the diameter typically remains smaller than 200 nm. The smallest fibrils are in the scale of so-called elementary fibrils, their diameter being typically in the range of 2-12 nm. The dimensions and size distribution of the fibrils may depend on the refining method and efficiency. Nanofibrillar cellulose may be characterized as a cellulose-based material, in which the median length of particles (fibrils or fibril bundles) is not greater than 50 µm, for example in the range of 1-50 µm, and the particle diameter is smaller than 1 µm, for example in the range of 2-500 nm. In case of native nanofibrillar cellulose, in an embodiment the average diameter of a fibril is in the range of 5-100 nm, for example in the range of 10-50 nm. Intact, unfibrillated microfibril units may be present in the nanofibrillar cellulose. In the context of this specification, the term "nanofibrillar cellulose" is not meant to encompass non-fibrillar, rod-shaped cellulose nanocrystals or whiskers.

The nomenclature relating to nanofibrillar cellulose is currently not uniform, and terms may be inconsistently used in the literature. For example, the following terms may have been used as synonyms for nanofibrillar cellulose: cellulose nanofiber (CNF), nanofibril cellulose, nanofibrillated cellulose (NFC), nanocellulose, nano-scale fibrillated cellulose, microfibrillar cellulose, cellulose microfibrils, microfibrillated cellulose (MFC), and fibril cellulose.

Nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose may also contain small amounts of other wood components, such as hemicellulose or lignin. The amount is dependent on the plant source.

Different grades of nanofibrillar cellulose may be categorized based on three main properties: (i) size distribution, length and diameter; (ii) chemical composition; and (iii) rheological properties. To fully describe a grade, the properties may be used in parallel. Examples of different grades may include native (or non-modified) NFC, oxidized NFC (high viscosity), oxidized NFC (low viscosity), carboxymethylated NFC and cationized NFC. Within these main grades, also sub-grades may exist, for example: extremely well fibrillated vs. moderately fibrillated, high degree of substitution vs. low, low viscosity vs. high viscosity, etc. The fibrillation technique and the chemical pre-modification may have an influence on the fibril size distribution. Typically, non-ionic grades may have a wider fibril diameter (for example in the range of 10-100 nm, or 10-50 nm), while the chemically modified grades may be thinner (for example in the range of 2-20 nm). The distributions of the fibril dimensions may be also narrower for the modified grades. Certain modifications, especially TEMPO oxidation, may yield shorter fibrils.

Depending on the raw material source, e.g. hardwood (HW) vs. softwood (SW) pulp, different polysaccharide compositions may be present in the final nanofibrillar cellulose product. Commonly, the non-ionic grades are prepared from bleached birch pulp, which may yield a high xylene content (25 % by weight). Modified grades may be prepared either from HW or SW pulps. In such modified grades, the hemicelluloses may also be modified together with the cellulose domain. The modification may not be homogeneous, i.e. some parts may be modified to a greater extent than others. Thus, a detailed chemical analysis may not be possible - the modified products are typically complex mixtures of different polysaccharide structures.

In an aqueous environment, a dispersion of cellulose nanofibers may form a viscoelastic hydrogel network. The gel may be formed at relatively low concentrations of, for example, 0.05-0.2% (w/w), dispersed and hydrated entangled fibrils. The viscoelasticity of the NFC hydrogel may be characterized, for example, by dynamic oscillatory rheological measurements. The nanofibrillar cellulose hydrogels may exhibit characteristic rheological properties. For example, they are shear-thinning or pseudoplastic materials, which means that their viscosity depends on the speed (or force) by which the material is deformed. When measuring the viscosity in a rotational rheometer, the shear-thinning behavior is seen as a decrease in viscosity with increasing shear rate. The hydrogels show plastic behavior, which means that a certain shear stress (force) is required before the material starts to flow readily. This critical shear stress is often called the yield stress. The yield stress can be determined from a steady state flow curve measured with a stress controlled rheometer. When the viscosity is plotted as function of applied shear stress, a dramatic decrease in viscosity can be seen after exceeding the critical shear stress. The zero shear viscosity and the yield stress may be the most important rheological parameters to describe the suspending power of the materials. These two parameters may separate the different grades quite clearly and thus may enable classification of the grades.

The dimensions of the fibrils or fibril bundles may be dependent on the raw material and the disintegration method. Mechanical disintegration of the cellulose raw material may be carried out with any suitable equipment such as a refiner, grinder, disperser, homogenizer, colloider, friction grinder, pin mill, rotor-rotor dispergator, ultrasound sonicator, fluidizer such as microfluidizer, macrofluidizer or fluidizer-type homogenizer. The disintegration treatment may be performed at conditions in which water is sufficiently present to prevent the formation of bonds between the fibers.

In an example, the disintegration is carried out by using a disperser having at least one rotor, blade or similar moving mechanical member, such as a rotor-rotor dispergator. One example of a rotor-rotor dispergator is an Atrex device.

Another example of a device suitable for disintegrating is a pin mill, such as a multi-peripheral pin mill. One example of such device is described in US 6202946 B1.

In an embodiment, the disintegrating is carried out by using a homogenizer.

In the context of this specification, the term "fibrillation" may generally refer to disintegrating fiber material mechanically by work applied to the particles, whereby cellulose fibrils are detached from the fibers or fiber fragments. The work may be based on various effects, such as grinding, crushing or shearing, or a combination of these, or another corresponding action that reduces the particle size. The energy taken by the refining work may normally be expressed in terms of energy per processed raw material quantity, in units of e.g. kWh/kg, MWh/ton, or units proportional to these. The expressions "disintegration" or "disintegration treatment" may be used interchangeably with "fibrillation". The fiber material dispersion that is subjected to fibrillation may be a mixture of fiber material and water (or an aqueous solution), also herein called "pulp". The fiber material dispersion may refer generally to whole fibers, parts (fragments) separated from them, fibril bundles, or fibrils mixed with water, and typically the aqueous fiber material dispersion is a mixture of such elements, in which the ratios between the components are dependent on the degree of processing or on the treatment stage, for example number of runs or "passes" through the treatment of the same batch of fiber material.

The disintegrated fibrous cellulosic raw material may be modified or nonmodified fibrous raw material. Modified fibrous raw material means raw material where the fibers are affected by a modification treatment so that cellulose nanofibrils are more easily detachable from the fibers. The modification may be performed to fibrous cellulosic raw material which exists as a suspension in a liquid, e.g. pulp.

The modification treatment to the fibers may be chemical or physical. In chemical modification, the chemical structure of cellulose molecule is changed by a chemical reaction ("derivatization" of cellulose), for example so that the length of the cellulose molecule is not affected but functional groups are added to β-D-glucopyranose units of the polymer. The chemical modification of cellulose may take place at a certain conversion degree, which is dependent on the dosage of reactants and the reaction conditions, and often it is not complete so that the cellulose will stay in solid form as fibrils and does not dissolve in water. In physical modification anionic, cationic, or nonionic substances or any combination of these may be physically adsorbed on cellulose surface. The modification treatment may also be enzymatic. The cellulose in the fibers may be particularly ionically charged after the modification, because the ionic charge of the cellulose may weaken the internal bonds of the fibers and may later facilitate the disintegration to nanofibrillar cellulose. The ionic charge may be achieved by chemical or physical modification of the cellulose. The fibers may have a higher anionic or cationic charge after the modification compared with the starting raw material. Commonly used chemical modification methods for making an anionic charge may include oxidation, where hydroxyl groups are oxidized to aldehydes and carboxyl groups, sulphonization and carboxymethylation. A cationic charge in turn may be created chemically by cationization by attaching a cationic group to the cellulose, such as a quaternary ammonium group.

In other words, the azido-modified nanofibrillar cellulose may comprise further modifications, such as chemical modifications. Chemically or physically modified nanofibrillar cellulose may be used as the raw material for preparing the azido-modified nanofibrillar cellulose. Alternatively, the azido-modified nanofibrillar cellulose may be prepared prior to further chemical or physical modification, e.g. by conjugating an azide-containing compound with alkenylated nanofibrillar cellulose and subsequently modifying the azido-modified nanofibrillar cellulose chemically or physically.

The nanofibrillar cellulose hydrogel may also be a mixture of the azido-modified nanofibrillar cellulose and one or more other nanofibrillar cellulose types or grades.

In an embodiment, the nanofibrillar cellulose comprises chemically modified nanofibrillar cellulose, such as anionically modified nanofibrillar cellulose or cationically modified nanofibrillar cellulose. In an embodiment, the nanofibrillar cellulose is anionically modified nanofibrillar cellulose. In an embodiment, the nanofibrillar cellulose is cationically modified nanofibrillar cellulose. In an embodiment, the anionically modified nanofibrillar cellulose is oxidized nanofibrillar cellulose. In an embodiment, the anionically modified nanofibrillar cellulose is sulphonized nanofibrillar cellulose. In an embodiment, the anionically modified nanofibrillar cellulose is carboxymethylated nanofibrillar cellulose.

The cellulose may be oxidized. In the oxidation of cellulose, primary hydroxyl groups of cellulose may be oxidized catalytically by a heterocyclic nitroxyl compound, for example 2,2,6,6-tetramethylpiperidinyl-1-oxy free radical, generally called "TEMPO". At least some of the primary hydroxyl groups (C6-hydroxyl groups) of the cellulosic β-D-glucopyranose units may be selectively oxidized to carboxylic groups. Some aldehyde groups may also be formed from the primary hydroxyl groups. The cellulose may be oxidized to a level having a carboxylic acid content in the oxidized cellulose in the range of 0.6-1.4 mmol COOH/ g pulp, or 0.8-1.2 mmol COOH / g pulp, for example to 1.0-1.2 mmol COOH/ g pulp, determined by conductometric titration. When the fibers of oxidized cellulose obtained in this manner are disintegrated in water, they may give a stable transparent dispersion of individualized cellulose fibrils, which may be, for example, of 3-5 nm in width.

The nanofibrillar cellulose may also be characterized by the average diameter (or width), or by the average diameter together with the viscosity, such as Brookfield viscosity or zero shear viscosity. In an embodiment, said nanofibrillar cellulose has a number average diameter of a fibril in the range of 1-100 nm. In an embodiment said nanofibrillar cellulose has a number average diameter of a fibril in the range of 1-50 nm. In an embodiment, said nanofibrillar cellulose has a number average diameter of a fibril in the range of 2-15 nm, such as TEMPO oxidized nanofibrillar cellulose. The diameter of a fibril may be determined using several techniques, such as by microscopy. Fibril thickness and width distribution may be measured by image analysis of the images from a field emission scanning electron microscope (FE-SEM), a transmission electron microscope (TEM), such as a cryogenic transmission electron microscope (cryo-TEM), or an atomic force microscope (AFM). In general, AFM and TEM may be well suited for nanofibrillar cellulose grades with narrow fibril diameter distribution.

The viscosity of the nanofibrillar cellulose may be measured using a rheometer. In an example, a rheometer viscosity of the nanofibrillar cellulose dispersion is measured at 22°C with a stress controlled rotational rheometer (AR-G2, TA Instruments, UK) equipped with a narrow gap vane geometry (the vane having a diameter of 28 mm and a length of 42 mm) in a cylindrical sample cup having a diameter of 30 mm. After loading the samples to the rheometer they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s-1 is exceeded. This method may be used for determining the zero-shear viscosity.

In one example the nanofibrillar cellulose, when dispersed in water, provides a zero shear viscosity ("plateau" of constant viscosity at small shearing stresses) in the range of 1000-100000 Pa·s, such as in the range of 5000- 50000 Pa·s, and a yield stress (shear stress where the shear thinning begins) in the range of 1-50 Pa, such as in the range of 3-15 Pa, determined by rotational rheometer at a consistency of 0.5% (w/w) by weight in aqueous medium.

The nanofibrillar cellulose may have a storage modulus in the range of 0.3 to 50 Pa, when dispersed to a concentration of 0.5 w% in water. For example, the storage modulus may be in the range of 1 to 20 Pa, or in the range of 2 to 10 Pa, when dispersed to a concentration of 0.5 w% in water.

Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry is used. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample. In a turbidity measurement method, a nanofibrillar cellulose sample may be diluted in water, to a concentration below the gel point of said nanofibrillar cellulose, and turbidity of the diluted sample may be measured. The concentration in which the turbidity of the nanofibrillar cellulose samples is measured may be 0.1%. HACH P2100 Turbidometer with a 50 ml measuring vessel may be used for turbidity measurements. The dry matter of the nanofibrillar cellulose sample is determined and 0.5 g of the sample, calculated as dry matter, may be loaded in the measuring vessel, which may be filled with tap water to 500 g and vigorously mixed by shaking for about 30 s. Without delay the aqueous mixture may be divided into 5 measuring vessels, which are inserted in the turbidometer. Three measurements on each vessel may be carried out. The mean value and standard deviation may be calculated from the obtained results, and the final result may be given as NTU units.

One way to characterize nanofibrillar cellulose is to define both the viscosity and the turbidity. Low turbidity may correlate with a small size of the fibrils, such as small diameter, as small fibrils scatter light poorly. In general as the fibrillation degree increases, the viscosity increases and at the same time the turbidity decreases. This may happen, however, until a certain point. When the fibrillation is further continued, the fibrils may finally begin to break and cannot form a strong network any more. Therefore, after this point, both the turbidity and the viscosity may begin to decrease.

In an example, the turbidity of anionic nanofibrillar cellulose is lower than 90 NTU, for example from 3 to 90 NTU, such as from 5 to 60, for example 8-40, measured at a consistency of 0.1% (w/w) in aqueous medium, and measured by nephelometry. In an example the turbidity of native nanofibrillar may be even over 200 NTU, for example from 10 to 220 NTU, such as from 20 to 200, for example 50-200 measured at a consistency of 0.1% (w/w) in aqueous medium, and measured by nephelometry. To characterize the nanofibrillar cellulose, these ranges may be combined with the viscosity ranges of the nanofibrillar cellulose.

The starting material for the preparation process may be nanofibrillar cellulose obtained or obtainable directly from the disintegration of some of the above-mentioned fibrous raw material and at a relatively low concentration homogeneously distributed in water due to the disintegration conditions. The starting material may be an aqueous gel at a concentration of 0.2-10%.

The term "azido-modified nanofibrillar cellulose" may be understood as referring to nanofibrillar cellulose chemically modified such that it has azido (-N₃) groups covalently bound thereto.

The azido-modified nanofibrillar cellulose of the nanofibrillar cellulose hydrogel comprises glucosyl units, one or more of which may be substituted. In the nanofibrillar cellulose hydrogel, the azido-modified nanofibrillar cellulose has a substituent represented by the formula -O-(CH₂)ₙ-S(O)ₘ-L₁-N₃, wherein n is in the range of 1 to 10; m is 0 or 1; and L₁ is a linker. The substituent is attached to a carbon of one or more glucosyl units of the azido-modified nanofibrillar cellulose. The substituent thus forms an ether bond to the carbon.

In an embodiment, n is 1, 2, 3, 4, 5, 6, 7, 8, 9 or 10. In an embodiment, n is in the range of 1 to 2, or in the range of 1 to 3, or in the range of 1 to 4, or in the range of 1 to 5, or in the range of 1 to 6, or in the range of 1 to 7, or in the range of 1 to 8. In an embodiment, n is in the range of 3 to 10.

Each glucosyl unit of the nanofibrillar cellulose comprises 6 carbon atoms numbered 1 to 6 according to the convention in the field. In unmodified nanofibrillar cellulose, carbons 2, 3 and 6 have hydroxyl groups attached to them. Each of these carbons may have a single hydroxyl group attached to them. The substituent may thus be attached to any one of these carbons, in place of the hydroxyl group. In other words, the substituent may be considered to replace the hydroxyl group attached to the carbon.

The term "a carbon of one or more glucosyl units" may be understood as referring to one or more carbons of one or more glucosyl units. In other words, one or more carbons of a single glucosyl unit may have the substituent according to one or more embodiments described in this specification attached thereto. Additionally or alternatively, one or more carbons of a plurality of glucosyl units may be substituted according to one or more embodiments of the substituent described in this specification. As a skilled person is well aware, the cellulose fibrils of the nanofibrillar cellulose, or fibril bundles derived from cellulose raw material, may contain cellulose molecules comprising chains of hundreds or thousands of glucosyl (typically β1,4-D-glucopyranosyl) units. Individual glucosyl units of a cellulose chain may therefore be substituted independently of other glucosyl units of the cellulose chain.

In this context, the term "substituent" refers to a moiety represented by the formula -O-(CH₂)ₙ-S(O)ₘ-L₁-N₃, wherein n is in the range of 1 to 10; m is 0 or 1; and L₁ is a linker. The substituent may be attached to the carbon (i.e. one or more carbons) in place of the hydroxyl group that would otherwise be attached to the carbon of the glucosyl unit. The hydroxyl group on the carbon (i.e. one or more carbons) of one or more glucosyl units of the azido-modified nanofibrillar cellulose may thus be replaced by the substituent. The substituent represented by the formula -O-(CH₂)ₙ-S(O)ₘ-L₁-N₃ as defined herein is covalently (directly) bound to the carbon; an ether bond is thus formed between the carbon and the substituent via the oxygen atom of the substituent.

In addition to the one or more glucosyl units, other saccharide units of the nanofibrillar cellulose have the substituent according to one or more embodiments described in this specification attached thereto. Depending e.g. on the raw material, nanofibrillar cellulose may also contain other wood components, such as hemicellulose and/or lignin. The hemicellulose of the azido-modified nanofibrillar cellulose may also be substituted. Hemicellulose of the azide-modified nanofibrillar cellulose may therefore also have the substituent according to one or more embodiments described in this specification attached to a carbon of one or more saccharide units of the hemicellulose, in a similar manner as to the azido-modified nanofibrillar cellulose. The one or more saccharide units of the hemicellulose may be xylosyl units, glucuronoxylosyl units, arabi-noxylosyl units, glucomannosyl units, xyloglucosyl units and/or any combinations or polymers thereof.

It may also be understood that in the azido-modified nanofibrillar cellulose, the substituents may be represented by a mixture of substituents according to one or more embodiments described in this specification. In other words, individual substituents of the azido-modified nanofibrillar cellulose or of a single chain of the cellulose of the azido-modified nanofibrillar cellulose may, at least in some embodiments, be independently selected from one or more embodiments of the substituent described in this specification. Two or more different substituents may be introduced into the azido-modified nanofibrillar cellulose on purpose, and/or they may be introduced e.g. by different chemical reactions occurring during the preparation of the azido-modified nanofibrillar cellulose. All substituents of the azido-modified nanofibrillar cellulose are therefore not necessarily represented by the same formula.

Furthermore, for example, the azido-modified nanofibrillar cellulose may also have an alkenyl (or allyl) substituent represented by the formula -O-(CH₂)ₙCH=CH₂ wherein n is in the range of 1 to 8, the alkenyl (or allyl) substituent being attached to a carbon of one or more glucosyl units of the azido-modified nanofibrillar cellulose, thus forming an ether bond to the carbon. While such an embodiment is typically not desirable, it may occur as a side product, when the alkenyl substituent has not reacted further.

In some embodiments, the sulfoxide structure (the group -S(O)-, i.e. -S(=O)-) may be present in the substituent, i.e. m may be 1. In other embodiments, the sulfoxide structure is not present, i.e. m is 0. It may also be understood that in the azido-modified nanofibrillar cellulose, the substituents may represent a mixture. Therefore, in some of the substituents of the azido-modified nanofibrillar cellulose or in a single chain of the cellulose of the azido-modified nanofibrillar cellulose, m may be 0 and in others m may be 1. While not to be bound by theory, the presence of the sulfoxide structure may depend e.g. on the reagents and/or the conditions used for preparing the azido-modified nanofibrillar cellulose. For example, in UV catalyzed (activated) reactions, the sulfoxide structure is not necessarily formed, while in chemically catalyzed (activated) reactions, the sulfoxide structure may be formed in a significant proportion of the substituents present in the azido-modified nanofibrillar cellulose.

In an embodiment, L₁ represents -CH₂-CH₂ (R₁) - NH-L₂-, wherein R₁ is absent or -COOH, and L₂ is a linker.

In other words, in the nanofibrillar cellulose hydrogel, the azido-modified nanofibrillar cellulose may have a substituent represented by the formula -O-(CH₂)ₙ-S(O)ₘ-CH₂-CH₂(R₁) -NH-L₂-N₃, wherein n, m, R₁ and L₂ are as defined in this specification, wherein the substituent is attached to a carbon of one or more glucosyl units of the azido-modified nanofibrillar cellulose. The substituent may thus form an ether bond to the carbon.

In an exemplary embodiment, the azido-modified nanofibrillar cellulose may be represented by the following formula:

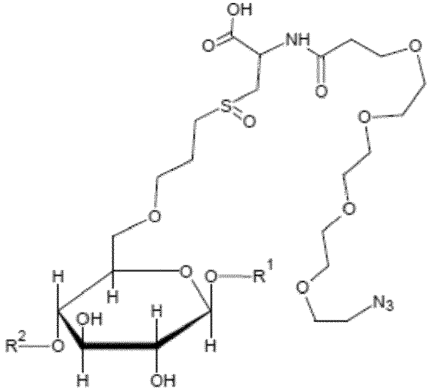

In this formula and in other formulae below containing them, R¹ and R² represent adjacent glucosyl units or chains of the nanofibrillar cellulose molecule joined together by glycoside links from carbon 1 and carbon 4 of the glycosyl unit.

In other words, in this embodiment, the substituent attached to carbon 6 of the β1,4-D-glucose unit of the azido-modified nanofibrillar cellulose is represented by the formula -O-(CH₂)ₙ-S(O)ₘ-L₁-N₃, wherein n is 3; m is 1; L₁ represents -CH₂-CH₂ (R₁) -NH-L₂-, wherein R₁ is -COOH, and L₂ represents C(O)-(CH₂-CH₂-O)ₒ-CH₂-CH₂-, wherein o is 4. In further embodiments of the formula, m may be 0; R₁ may be absent; and/or o may be 0, 1 or greater. As depicted, the substituent forms an ether bond to the carbon 6 via the oxygen atom of the substituent.

A "linker" in the context of this specification, including L₁ and/or L₂, may comprise one or more linker groups or moieties and/or one or more spacer groups. The linker group may, in principle, be any linker group that can be incorporated in the azido-modified nanofibrillar cellulose according to one or more embodiments described in this specification. A large number of different linkers are known in the art and may be commercially available. It may also comprise one or more groups formed by a reaction between two functional groups. A skilled person will realize that various different chemistries may be utilized, and thus a variety of different functional groups may be reacted to form groups or moieties comprised by L₁ and/or L₂, for example sulfhydryl, amino, alkenyl, alkynyl, azidyl, aldehyde, carboxyl, maleimidyl, succinimidyl and/or hydroxylamino groups. A skilled person is capable of selecting the functional groups so that they may react in certain conditions.

The linker may be hydrophilic. A hydrophilic linker may have the utility that it may be relatively well soluble in an aqueous solution. For example, the linker may be a peptide linker, for example a peptide linker from 2 to 5 amino acids in length. The length of the linker is not particularly limited and may be selected e.g. depending on the size of the ligand. The linker and its length may be selected so as to reduce steric hindrances, optimize solubility of the azido-modified or ligand-modified nanofibrillar cellulose in aqueous solutions and/or optimize various properties of the nanofibrillar cellulose hydrogel.

The linker, including L₁ and/or L₂, may comprise or be, for example, any one of the following groups or moieties:
(a) other polyalkylene glycol or a derivative thereof, including a polypropylene glycol ho-mopolymers and copolymer of ethylene glycol with propylene glycol;
(b) a peptide or a derivative thereof, including a peptide or derivative thereof of the formula - (AA)ₙ-, wherein AA is any amino acid and n is an integer between 1 and about 100;
(c) an oligosaccharide or a polyol;
(d) a starch, a dextrine, a dextran or a dextran derivative, including dextran sulfate, cross linked dextrin, and carboxymethyl dextrin;
(e) heparin or a fragment of heparin;
(f) polyvinyl alcohol or polyvinyl ethyl ether;
(g) polyvinylpyrrolidone;
(h) α,β-poly[(2-hydroxyethyl)-DL-aspartamide;
(i) a polyoxyethylated polyol; or
(j) an alkane, a substituted alkane, an alkene, a substituted alkene, an alkyne, a substituted alkyne, or a derivative thereof.

Examples of suitable linkers or linker moieties are e.g. the spacer moieties described in WO2004100997, for example the poly(ethylene glycol) moieties described therein.

In an embodiment, L₂ represents C(O)-(CH₂-CH₂-O)ₒ-CH₂-CH₂-, wherein o is 0 or greater. In an embodiment, o may be 1 or greater. In an embodiment, o may be in the range of 0 to 100 or 1 to 100, or in the range of 0 to 20 or 1 to 20. o may be 1, 2, 3, 4, 5, 6, 7, 8, 9, or 10. This type of linker may be relatively biocompatible, hydrophilic and flexible. o, i.e. the number of (CH₂-CH₂-O) units in L₂, may be selected depending e.g. on the size of the ligand.

Any one of the carbons of the glucosyl units of the nanofibrillar cellulose that would, without the azido modification (substituent) be directly linked to a primary or secondary hydroxyl group, may be modified such that the substituent is attached to a carbon in place of the primary or secondary hydroxyl group. One or more of the carbons 2, 3, and 6 of one or more β1,4-D-glucopyranosyl units of the nanofibrillar cellulose may be modified as described in this specification. In other words, one or more of the carbons 2, 3, and 6 of one or more β1,4-D-glucopyranosyl units of the nanofibrillar cellulose may have a substituent according to one or more embodiments described in this specification attached thereto.

It may also be possible to selectively direct the modification to carbon 6 (C6), i.e. the primary hydroxyl group of D-glucopyranosyl residues in cellulose. In other words, at least one or more of the one or more glucosyl units may be β1,4-D-glucopyranosyl units, and the carbon of the one of more β1,4-D-glucopyranosyl units to which the substituent is attached to may be carbon 6.

The azido-modified nanofibrillar cellulose may have a degree of substitution (DS) of at least about 0.0001, at least about 0.001, at least about 0.01, or at least about 0.05, or at least about 0.1, at least about 0.2, at least about 0.3, at least about 0.4, at least about 0.5, at least about 0.6, at least about 0.7, at least about 0.8, at least about 0.9, or about 1. In an embodiment, DS is about 0.09. In an embodiment, DS is about 0.06. In this context, the DS may specifically refer to a DS by a substituent according to one or more embodiments in this specification. The term "degree of substitution" may be understood as referring to the number or average number of the substituent groups attached per glucosyl unit of the azido-modified or ligand-modified nanofibrillar cellulose. For calculation of molar amount of available azido groups per gram of dry nanofibrillar cellulose, a structural unit of nanofibrillar cellulose may be understood as having a mass of about 162.1 g/mol, which corresponds to the mass of a glucose residue, in other words an anhydroglucose. Thus, 1 mol of nanofibrillar cellulose may be understood as having a mass of about 162.1 g, and 1 g of nanofibrillar cellulose may be understood as having a molar amount of about 6.17 mmol. Thus, the degree of substitution of 0.01 may correspond to 10 mmol of the azido groups per 162.1 g of dry nanofibrillar cellulose, or 0.06 mmol/g.

The DS may be such that the content or number of the azido groups is in excess of the content or number of the ligand(s) to be linked to the azido-modified nanofibrillar cellulose.

A kit comprising the nanofibrillar cellulose hydrogel according to one or more embodiments described in this specification is also disclosed. The kit may further comprise instructions for use.

A solid support comprising or containing the nanofibrillar cellulose hydrogel according to one or more embodiments described in this specification is also disclosed. The solid support may be e.g. a multiwell plate, a vessel, a bioreactor, a scaffold, or a 3-D microfluidic cell culture chip (organ-on-a-chip). The solid support may be suitable for culturing cells and/or tissues. The solid support has a recess for receiving or containing the nanofibrillar cellulose hydrogel (azido-modified and/or ligand-modified).

A kit for preparing a ligand having a cyclic or acyclic alkyne group is also disclosed, the kit comprising a linker compound conjugable to a ligand and having a cyclic alkyne group.

The kit further comprises nanofibrillar cellulose hydrogel comprising azido-modified nanofibrillar cellulose according to one or more embodiments described in this specification.

The kit may further comprise the solid support according to one or more embodiments described in this specification.

The kit or the solid support may further comprise e.g. a reaction buffer and/or additional reagents. For example, the kit may comprise the linker compound conjugable to the ligand and having the cyclic alkyne group in a dry form, so the kit may comprise an aqueous solution for reconstituting the dry linker compound. The kit or solid support may further comprise a cell culture medium.

The kit may further comprise a ligand, although not necessarily. The ligand may be obtained separately.

The kit may further comprise a ligand having a cyclic or acyclic alkyne group. Alternatively or additionally, the kit may further comprise a linker compound conjugable to a ligand, which linker compound may have a cyclic or acyclic alkyne group. The linker compound conjugable to a ligand may be any suitable linker compound described in this specification. The instructions for use may comprise instructions for preparing the nanofibrillar cellulose hydrogel comprising ligand-modified nanofibrillar cellulose according to one or more embodiments described in this specification.

In the context of this specification, the term "ligand" may be understood as referring to a molecule that may form a complex with a biomolecule to serve a biological purpose. The ligand may be a biomolecule, e.g. a peptide, a protein (for example a glycoprotein), a glycan, or any mixture or combination thereof. The ligand may be capable of forming a complex with a molecule of a cell, for example a cell that may be suitable for being cultured in contact with the nanofibrillar cellulose hydrogel. The ligand may be capable of forming a complex with a molecule on the surface of the cell. The ligand may be capable of promoting the attachment/adhesion of cells to the nanofibrillar cellulose hydrogel and/or the growth of cells, for example a growth factor, an adhesion molecule or a ligand capable of binding to an adhesion molecule. The size of the ligand is not particularly limited. The term "ligand" or "a ligand" may also encompass one or more ligands, e.g. a mixture of ligands.

The ligand may be or comprise, for example, an extracellular matrix (ECM) component, a lectin, S-type lectin, C-type lectin, P-type lectin, I-type lectin, a galectin, galectin-1, galectin-3, a galectin ligand, a lipid, a glycolipid, a glycoside, a galactoside, a pro-teoglycan, an oligosaccharide, a polysaccharide, a gly-cosamino-glycan, heparin, heparan sulfate, chondrotin, chondroitin sulfate, keratan sulfate, hyaluronan, transforming growth factor β1 (TGF-β1), basic fibroblast growth factor (bFGF), leukemia inhibitory factor (LIF), an integrin (for example, αV, β1, β5 α5, or α6 ntegrin), α6β1 integrin, α3β1 integrin, α1β1 or α2β1 integrin, an integrin ligand, talin, vinculin, kindlin, a cadherin, epithelial (E) cadherin, neural (N) cadher-in, vascular endothelial (VE) cadherin, a cadherin ligand, a selectin, a selectin ligand, a laminin, laminin-511, lamini-111, laminin-332, laminin-521, a laminin ligand, nido-gen, fibronectin, fibronectin type I domain, fibronectin type II domain, fibronectin type II domain, an RGD-peptide, an RGD adhesive peptide (e.g. GRGDSPC, SEQ ID No: 1), vitronectin, vitronectin oligopeptide KGG-PQVTRGDVFTMP (SEQ ID No: 2), a collagen, collagen type I, collagen type IV, or a domain, a fragment, or a modification thereof.

The term "ligand having a cyclic (or acyclic) alkyne group" may be understood as referring to any ligand described in this specification, to which a cyclic (or acyclic) alkyne group is covalently bound, either directly or via one or more linkers and/or spacers.

The cyclic or acyclic alkyne group may be capable of reacting with the azido groups of the azido-modified nanofibrillar cellulose. Acyclic alkyne groups may require a catalyst to react efficiently, for example a copper(I) catalyst. Cyclic alkyne groups may react with azido groups in a biorthogonal reaction, i.e. cycloaddition reaction in the absence of an exogenous metal (e.g. copper) catalyst. Thus, with cyclic alkyne groups, there is no need for an exogenous metal catalyst, so no potentially cytotoxic metal ions will have to be included as a catalyst in the reaction.

The cyclic alkyne group may be DBCO (dibenzylcyclooctyne), OCT (cyclooctyne), MOFO (monofluorinated cyclooctyne), ALO (aryl-less octyne), DIFO (difluorocyclooctyne), DIFO2 (difluorocyclooctyne), DIFO3 (difluorocyclooctyne), DIMAC (dimethoxyazacyclooctyne), DIBO (dibenzocyclooctyne), DIBAC (dibenzoazacyclooctyne), BARAC (biarylazacyclooctynone), BCN (bicyclononyne), Sondheimer diyne, TMDIBO (2,3,6,7-tetramethoxy-DIBO), S-DIBO (sulfonylated DIBO), COMBO (carboxymethyl-monobenzocyclooctyne), PYRROC (pyrrolocyclooctyne), or a modification or analog thereof.

The cyclic alkyne group may be selected from the group consisting of DBCO, OCT, MOFO, ALO, DIFO, DIFO2, DIFO3, DIMAC, DIBO, DIBAC, BARAC, BCN, Sondheimer diyne, TMDIBO, S-DIBO, COMBO, PYRROC, and a modification or analog thereof.

The structures of the above cyclic alkyne groups are shown below in Table 1. A skilled person will understand that any of the cyclic alkyne groups described herein may be bound to a ligand and/or to a linker via a suitable atom or group of the cyclic alkyne group, for example the N atom of DBCO or the O atom of DIBO.

**Table 1. Structures of cyclic alkyne groups.**

| | |
|---|---|
| DBCO (dibenzylcyclooctyne) | 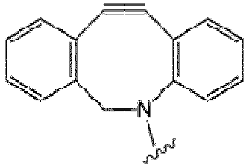 |
| OCT (cyclooctyne) | 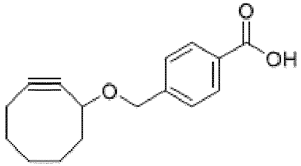 |
| ALO (aryl-less octyne) | 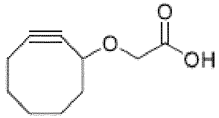 |
| MOFO (monofluorinated cyclooctyne) | 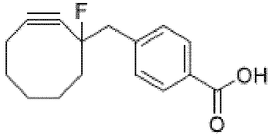 |
| DIFO (difluorocyclooctyne) | 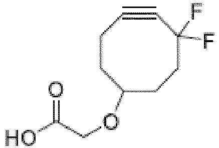 |
| DIFO2 | 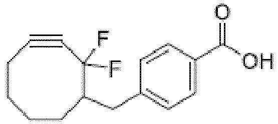 |
| DIFO3 | 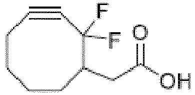 |
| DIMAC (dimethoxyazacyclooctyne) | 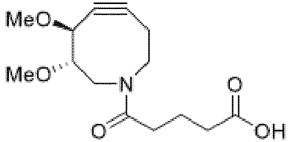 |
| DIBO (dibenzocyclooctyne) | 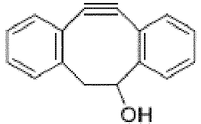 |
| DIBAC (dibenzoazacyclooctyne) | 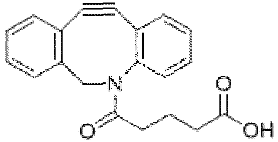 |
| BARAC (biarylazacyclooctynone) | 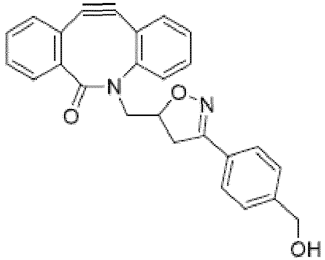 |
| BCN (bicyclononyne) | 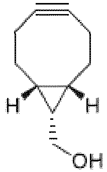 |
| Sondheimer diyne | 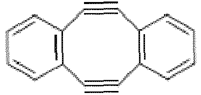 |
| TMDIBO (2,3,6,7-tetramethoxy-DIBO) | 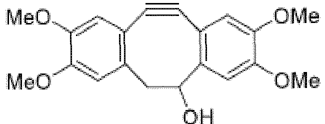 |
| S-DIBO (sulfonylated DIBO) | 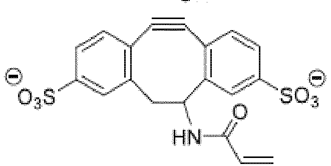 |
| COMBO (carboxymethylmonobenzocyclooctyne) | 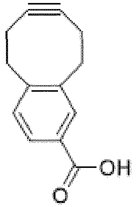 |
| PYRROC (pyrrolocyclooctyne) | 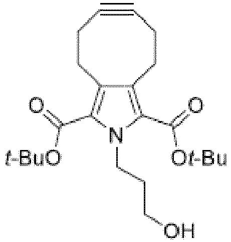 |

Various modifications and analogs of these cyclic alkynes may also be contemplated or developed.

The linker compound conjugable to a ligand may comprise one or more linker groups or moieties. It may also comprise one or more groups formed by a reaction between two functional groups. A skilled person will realize that various different chemistries may be utilized when preparing the compound, and thus a variety of different functional groups may be reacted to form groups comprised by the linker compound conjugable to a ligand. Furthermore, the linker compound conjugable to a ligand may comprise one or more functional groups conjugable to the ligand; the functional group(s) may be selected depending e.g. on the ligand. For example, the linker compound conjugable to a ligand may comprise at least one of sulfhydryl, amino, alkenyl, alkynyl, azidyl, aldehyde, carboxyl, maleimidyl, succinimidyl, hydroxylamino groups. The linker compound conjugable to a ligand may comprise N-hydroxysuccinimidyl (NHS) or N-hydroxysulfosuccinimidyl (sulfo-NHS) ester. For example, the linker compound conjugable to a ligand may comprise or be NHS-sulfo-DBCO (dibenzocyclooctyne-sulfo-N-hydroxysuccinimidyl ester) or NHS-DBCO (dibenzocyclooctyne-N-hydroxysuccinimidyl ester), wherein the NHS and DBCO may optionally be linked via a linker or spacer group. In this specification, "succinimidyl" may refer to N-hydroxysuccinimidyl (NHS) or N-hydroxysul-fosuccinimidyl (sulfo-NHS). Such compounds may react with a primary amino group of the ligand. Various other amino reactive functional groups may also be contemplated, for example imidoesters.

A method for preparing the nanofibrillar cellulose hydrogel comprising the azido-modified nanofibrillar cellulose according to one or more embodiments described in this specification is disclosed. The method comprises
alkenylating of one or more glucosyl units of nanofibrillar cellulose with an alkenylating agent to obtain alkenylated nanofibrillar cellulose, and
conjugating an azido-containing compound with the alkenylated nanofibrillar cellulose, thereby obtaining the nanofibrillar cellulose hydrogel comprising the azido-modified nanofibrillar cellulose.

The method may comprise providing a nanofibrillar cellulose hydrogel comprising the nanofibrillar cellulose prior to alkenylating.

The alkenylating agent has a structure represented by the formula X-(CH₂)ₙCH=CH₂, wherein n is in the range from 1 to 8, and X is Br, Cl, or I.

In an embodiment, n is 1, 2, 3, 4, 5, 6, 7 or 8. In an embodiment, n is in the range of 1 to 2, or in the range of 1 to 3, or in the range of 1 to 4, or in the range of 1 to 5, or in the range of 1 to 6, or in the range of 1 to 7.

In an embodiment, the alkenylating agent is allyl bromide. When allyl bromide is used as the alkenylating agent, the alkenylated (i.e. allylated) nanofibrillar cellulose may be represented by the following formula:

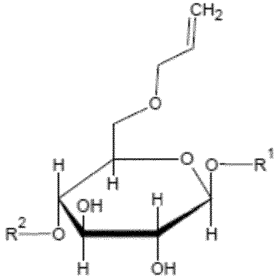

In this formula and in other formulae below containing them, R¹ and R² represent adjacent glucosyl units or chains of the nanofibrillar cellulose molecule joined together by glycoside links from carbon 1 and carbon 4 of the glycosyl unit.

It may also be possible to selectively direct the alkenylation to the hydroxyl group of carbon 6 (C6), i.e. the primary hydroxyl group of D-glucopyranosyl residues in cellulose. In other words, at least one or more of the one or more glucosyl units may be β1,4-D-glucopyranosyl units, and the hydroxyl group on the carbon of the one of more β1,4-D-glucopyranosyl units that is alkenylated may be the hydroxyl group on carbon 6.

Conjugating an azido-containing compound with the alkenylated nanofibrillar cellulose may be done in one or more steps.

The method may comprise reacting a thiol group-containing compound with the alkenylated nanofibrillar cellulose, wherein the thiol group-containing compound further has an azido group, thereby obtaining the nanofibrillar cellulose hydrogel comprising the azido-modified nanofibrillar cellulose. In other words, the azido-containing compound may be a thiol group-containing compound which further has an azido group.

The method may comprise reacting a thiol group-containing compound with the alkenylated nanofibrillar cellulose, wherein the thiol group-containing compound further has an amine group, thereby obtaining an amino-modified nanofibrillar cellulose, and reacting a compound having a functional group capable of reacting with the amino group with the amino-modified nanofibrillar cellulose, wherein the compound having the functional group further has an azido group, thereby obtaining the nanofibrillar cellulose hydrogel comprising the azido-modified nanofibrillar cellulose.

The thiol group-containing compound may be cysteine, cysteamine, or a combination or a mixture thereof. When allyl bromide is used as the alkenylating agent and cysteine as the thiol group-containing compound, the amino-modified nanofibrillar cellulose may be represented by the following formula:

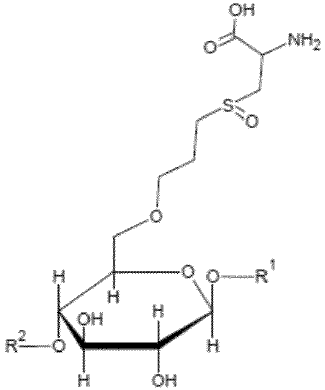

The thiol group-containing compound may be reacted with the alkenylated nanofibrillar cellulose in the presence of a radical initiator. The radical initiator is capable of catalyzing the reaction between the alkenyl group(s) of the alkenylated nanofibrillar cellulose and the thiol (sulfhydryl) group. In the context of this specification, "radical initiator" may be understood as referring to an agent capable of producing radical species under mild conditions and promote radical reactions. The term "radical initiator" may also refer to UV (ultraviolet) light. UV light irradiation is capable of generating radicals, e.g. in the presence of a suitable photoinitiator. Suitable radical initiators may include, but are not limited to, inorganic peroxides such as ammonium persulfate or potassium persulfate, organic peroxides, and UV light.

When allyl bromide is used as the alkenylating agent, cysteine as the thiol group-containing compound, and NHS-PEG4-azide (N-hydroxysuccinimide ester- PEG-azide linker with 4 -(CH₂-CH₂-O)- units in the PEG moiety) is used as the compound having a functional group capable of reacting with the amino group, the azido-modified nanofibrillar cellulose may be represented by the following formula:

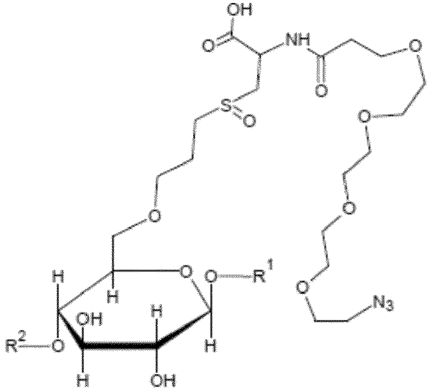

In other words, in this embodiment, the substituent is in place of, i.e. replaces, the hydroxyl group on a carbon in carbon 6 of the β1,4-D-glucose unit of the azido-modified nanofibrillar cellulose. The substituent is represented by the formula -O-(CH₂)ₙ-S(O)ₘ-L₁-N₃, wherein n is 3; m is 1; L₁ represents -CH₂-CH₂(R₁)-NH-L₂-, wherein R₁ is -COOH, and L₂ represents C(O)-(CH₂-CH₂-O) ₒ-CH₂-CH₂-, wherein o is 4. In further embodiments of the formula, m may be 0; R₁ may be absent; and/or o may be 0, 1 or greater.

The method, including all steps thereof, may be performed in an aqueous solution. A suitable aqueous solution may be e.g. an aqueous buffer solution, which may have a pH of about 6 to 8.

The nanofibrillar cellulose hydrogel to be modified may be diluted to a desired consistency for performing the method. Subsequently, the nanofibrillar cellulose hydrogel obtainable, comprising the azido-modified nanofibrillar cellulose, may be concentrated to a desired consistency for further use. The hydrogel may be concentrated e.g. by centrifuging or by filtering. If desired, the nanofibrillar cellulose hydrogel may be washed by diluting it in water or an aqueous solution and then concentrating.

The azido-containing compound may be directly reacted with the alkenylated nanofibrillar cellulose, thereby obtaining the nanofibrillar cellulose hydrogel comprising the azido-modified nanofibrillar cellulose. In such embodiments, the azido-containing compound may further have a thiol group. Further ways or routes to obtain the azido-modified nanofibrillar cellulose can also be contemplated.

A nanofibrillar cellulose hydrogel comprising ligand-modified nanofibrillar cellulose is disclosed, wherein the ligand-modified nanofibrillar cellulose has one or more ligands covalently bound thereto. The one or more ligands may comprise at least one of a protein, a peptide, a glycan or a nanofibrillar cellulose molecule.

The ligand-modified nanofibrillar cellulose may have one or more ligands covalently bound thereto via a group formed by a reaction between an azido group and a cyclic alkyne group. The group may be a triazole group, for example a 1,2,3-triazole group. The exact structure of the triazole group formed may depend on the structure of the cyclic alkyne group. The 1,2,3-triazolyl may thus be a group formed by click conjugation comprising a triazole moiety. Click conjugation should be understood as referring to a reaction between an azide and an alkyne yielding a covalent product - 1,5-disubstituted 1,2,3-triazole - such as copper(I)-catalysed azide-alkyne cycloaddition reaction (CuAAC). Click conjugation may also refer to copper-free click chemistry, such as a reaction between an azide and a cyclic alkyne group, such as dibenzocyclooctyl (DBCO). "1,2,3-triazolyl" may thus also refer to a group formed by a reaction between an azide and a cyclic alkyne group, such as DBCO, wherein the group comprises a 1,2,3-triazole moiety.

The cyclic alkyne group may be any cyclic alkyne group described in this specification. The cyclic alkyne group may be DBCO, OCT, MOFO, ALO, DIFO, DIFO2, DIFO3, DIMAC, DIBO, DIBAC, BARAC, BCN, Sondheimer diyne, TMDIBO, S-DIBO, COMBO, or PYRROC.

The ligand-modified nanofibrillar cellulose has a substituent represented by the formula -O-(CH₂)ₙ-S(O)ₘ-L₁-D, wherein n is in the range of 1 to 10; m is 0 or 1; L₁ is a linker; and D represents the ligand covalently bound to the linker; wherein the substituent is attached to a carbon of one or more glucosyl units of the ligand-modified nanofibrillar cellulose. The substituent thus forms an ether bond to the carbon. D represents the ligand and a triazole group formed by a reaction between the azido group of the nanofibrillar cellulose of the nanofibrillar cellulose hydrogel according to one or more embodiments described in this specification and a cyclic or acyclic alkyne group of the ligand.

In an embodiment, n is 1, 2, 3, 4, 5, 6, 7, 8, 9 or 10. In an embodiment, n is in the range of 1 to 2, or in the range of 1 to 3, or in the range of 1 to 4, or in the range of 1 to 5, or in the range of 1 to 6, or in the range of 1 to 7, or in the range of 1 to 8. In an embodiment, n is in the range of 3 to 10.

At least one or more of the one or more glucosyl units may be β1,4-D-glucopyranosyl units, and the carbon of the one of more β1,4-D-glucopyranosyl units having the substituent attached thereto may be carbon 6.

The carbon may, in some embodiments, be carbon 6. In other words, at least one or more of the one or more glucosyl units may be β1,4-D-glucopyranosyl units, and the carbon of the one of more β1,4-D-glucopyranosyl units to which the substituent is attached may be carbon 6. The substituent in carbon 6 may not significantly interfere with enzymatic degradation of the nanofibrillar cellulose hydrogel.

In an embodiment, the ligand-modified nanofibrillar cellulose has a substituent represented by the formula -O-(CH₂)ₙ-S(O)ₘ-L₁-D, wherein n is in the range of 1 to 10; m is 0 or 1; L₁ is absent or a linker; and D represents the ligand; wherein the substituent is attached to a carbon of one or more glucosyl units of the ligand-modified nanofibrillar cellulose. This embodiment may be prepared, for example, by reacting a ligand having a thiol group, for example a peptide or a protein ligand having a thiol group, directly with alkenylated nanofibrillar cellulose. The carbon may, in some embodiments, be carbon 6.

In an embodiment, n is 1, 2, 3, 4, 5, 6, 7, 8, 9 or 10. In an embodiment, n is in the range of 1 to 2, or in the range of 1 to 3, or in the range of 1 to 4, or in the range of 1 to 5, or in the range of 1 to 6, or in the range of 1 to 7, or in the range of 1 to 8. In an embodiment, n is in the range of 3 to 10.

In an embodiment, L₁ represents -CH₂-CH₂ (R₁) - NH-L₂-, wherein R₁ is absent or -COOH, and L₂ is a linker.

In an embodiment, L₂ represents C(O)-(CH₂-CH₂-O)ₒ-CH₂-CH₂-, wherein o is 0 or greater. In an embodiment, o may be 1 or greater. In an embodiment, o may be in the range of 0 to 100 or 1 to 100, or in the range of 0 to 20 or 1 to 20.

The ligand-modified nanofibrillar cellulose may have a degree of substitution (DS) of at least about 0.0001, at least about 0.001, at least about 0.01, or at least about 0.05, or at least about 0.1, at least about 0.2, at least about 0.3, at least about 0.4, at least about 0.5, at least about 0.6, at least about 0.7, at least about 0.8, at least about 0.9, or about 1. In an embodiment, DS is about 0.09. In an embodiment, DS is about 0.06. In this context, the DS may specifically refer to a DS by a substituent according to one or more embodiments in this specification comprising the ligand (or one or more ligands).

In an embodiment, the one or more ligands comprise at least one of a protein, a peptide, or a glycan.

The one or more ligands may comprise a nanofibrillar cellulose molecule. In other words, a second nanofibrillar cellulose molecule may be covalently bound to the ligand-modified nanofibrillar cellulose or to the ligand-modified nanofibrillar cellulose molecule. The ligand-modified nanofibrillar cellulose may therefore be cross-linked. Such a cross-linked ligand-modified nanofibrillar cellulose may be more stable and/or have desirable properties, for example viscosity or stiffness. The cross-linked nanofibrillar cellulose may be suitable e.g. for controlled release of active pharmaceutical ingredients or for 3D printing.

Two or more ligands selected from a protein, a peptide, a glycan, a nanofibrillar cellulose molecule, or any mixture or combination thereof, may be covalently bound to the ligand-modified nanofibrillar cellulose. Further, other ligands may additionally be covalently bound to the ligand-modified nanofibrillar cellulose. Various types of ligands may be contemplated, for example any ligand described in this specification.

A method for preparing a nanofibrillar cellulose hydrogel comprising ligand-modified nanofibrillar cellulose according to one or more embodiments described in this specification is disclosed. The method may comprise contacting the nanofibrillar cellulose hydrogel comprising the azido-modified nanofibrillar cellulose according to one or more embodiments described in this specification with a ligand having a cyclic or acyclic alkyne group.

The method may comprise providing the nanofibrillar cellulose hydrogel comprising the azido-modified nanofibrillar cellulose prior to contacting it with the ligand having the cyclic or acyclic alkyne group.

The ligand as such may have a cyclic or acyclic alkyne group, for example a synthetic ligand which has been prepared such that it has a cyclic or acyclic alkyne group. In cases in which the ligand does not already have a cyclic or acyclic alkyne group, one may be covalently linked, i.e. conjugated, thereto. This may be referred to as activating the ligand. The method may therefore comprise conjugating a linker compound having a cyclic or acyclic alkyne group to the ligand, thereby obtaining the ligand having the cyclic alkyne group, and contacting the azido-modified nanofibrillar cellulose hydrogel with the ligand having the cyclic or acyclic alkyne group. One or more linker compounds may be conjugated to the ligand, such that the cyclic or acyclic alkyne group is covalently bound to the ligand via one or more linkers or linker groups.

One or more ligands having cyclic or acyclic alkyne groups, e.g. a mixture of ligands, may be contacted with the nanofibrillar cellulose hydrogel comprising the azido-modified nanofibrillar cellulose.

The method, including all steps thereof, may be performed in an aqueous solution. A suitable aqueous solution may be e.g. an aqueous buffer solution, which may have a pH of about 6 to 8.

The nanofibrillar cellulose hydrogel to be prepared may be diluted to a desired consistency for performing the method. Subsequently, the nanofibrillar cellulose hydrogel obtainable, comprising the ligand-modified nanofibrillar cellulose, may be concentrated to a desired consistency for further use. The hydrogel may be concentrated e.g. by centrifuging or by filtering. If desired, the nanofibrillar cellulose hydrogel may be washed by diluting it in water or an aqueous solution and then concentrating.

The number or content of the azido groups, or the DS, may be in excess with respect to the amount of the ligand(s) or otherwise such that different numbers or amounts of ligands may be linked to the azido-modified nanofibrillar cellulose. This may allow e.g. preparing concentration series of the ligand, i.e. NFC hydrogels comprising ligand-modified nanofibrillar cellulose with different numbers or amounts of the ligand.

Use of the azido-modified or ligand-modified nanofibrillar cellulose hydrogel according to one or more embodiments described in this specification or use of the solid support according to one or more embodiments described in this specification for maintaining, transporting, isolating, culturing, propagating, passaging, differentiating or transplanting of cells or tissues is disclosed.

Use of the azido-modified or ligand modified nanofibrillar cellulose hydrogel according to one or more embodiments described in this specification for 3D printing is disclosed.

Use of the azido-modified or ligand-modified nanofibrillar cellulose hydrogel according to one or more embodiments described in this specification or use of the solid support according to one or more embodiments described in this specification for improving the adhesion, maintenance, transport, isolation, culture, propagation, passaging, differentiation or transplanting of cells, tissues, organoids or organs is also disclosed.

A method for maintaining, transporting, isolating, culturing, propagating, passaging, differentiating or transplanting of cells, tissues, organoids or organs is disclosed. The method may comprise contacting the cells, tissues, organoids or organs with the azido-modified or ligand-modified nanofibrillar cellulose hydrogel according to one or more embodiments described in this specification or with the solid support according to one or more embodiments described in this specification.

In the context of the uses or the methods for maintaining, transporting, isolating, culturing, propagating, passaging, differentiating or transplanting of cells or tissues, the cells may be any cells described in this specification, such as pluripotent stem cells, for example iPS cells. The amount of the ligand in the ligand-modified nanofibrillar cellulose hydrogel may be at least about 0.001 µg/ml by the volume of the growth medium in which the cells or tissues are maintained, transported, isolated, cultured, propagated, passaged, differentiated or transplanted. The growth medium may include the ligand-modified nanofibrillar cellulose hydrogel and optionally a second medium, for example a medium containing nutrients. Suitable second media may include e.g. various liquid media for cell and/or tissue culture. For example, the amount of the ligand in the ligand-modified nanofibrillar cellulose hydrogel may be at least about 0.01 µg/ml or at least about 0.1 µg/ml. The amount of the ligand in the ligand-modified nanofibrillar cellulose hydrogel may be up to about 500 µg/ml, or up to about 1 mg/ml, or up to about 10 mg/ml. For example, a well suited amount of the ligand in the ligand-modified nanofibrillar cellulose hydrogel may be 1 - 500 µg/ml by the volume of the growth medium, or 5 - 100 µg/ml.

In an embodiment of the uses or of the method, the azido-modified or ligand-modified nanofibrillar cellulose hydrogel or the solid support is used as a 3D culture matrix.

In an embodiment of the uses or of the method, the the azido-modified or ligand-modified nanofibrillar cellulose hydrogel or the solid support is used for the growth of an organ or an organoid. For example, a solid support that is a 3D microfluidic cell culture chip may be used for the growth of an organ or an organoid.

For example, in vitro generation of human intestinal organoids (HIO) from human pluripotent stem cell (hPSC) spheroids by supporting intestinal spheroid survival, expansion and epithelial differentiation into HIOs may require the presence of RGD adhesive peptide (GRGDSPC, SEQ ID No. 1) in the 3D culture matrix.

The interaction of cells with laminin in the nucleus pulposus (NP) region of the intervertebral disc (IVD) may promote cell attachment and biosynthesis. The incorporation of laminin type 111 (LM111) into the 3D cell culture matrix may be beneficial for promoting NP cell survival and phenotype.

Two key elements of the liver cell niche are laminin 521 and laminin 111. The human embryonic stem cell (hESC) organization, function, and differentiation to hepatocytes may improve significantly in the presence of laminin 521 and laminin 111.

### EXAMPLES

Reference will now be made in detail to various embodiments, an example of which is illustrated in the accompanying drawing.

The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

### EXAMPLE 1 - Generation of a nanofibrillar cellulose hydrogel comprising azido-modified nanofibrillar cellulose

In the following examples, the term "GrowDex" or "GrowDex^{®}" refers to nanofibrillar cellulose hydrogel. The GrowDex used in these examples is native nanofibrillar cellulose hydrogel.

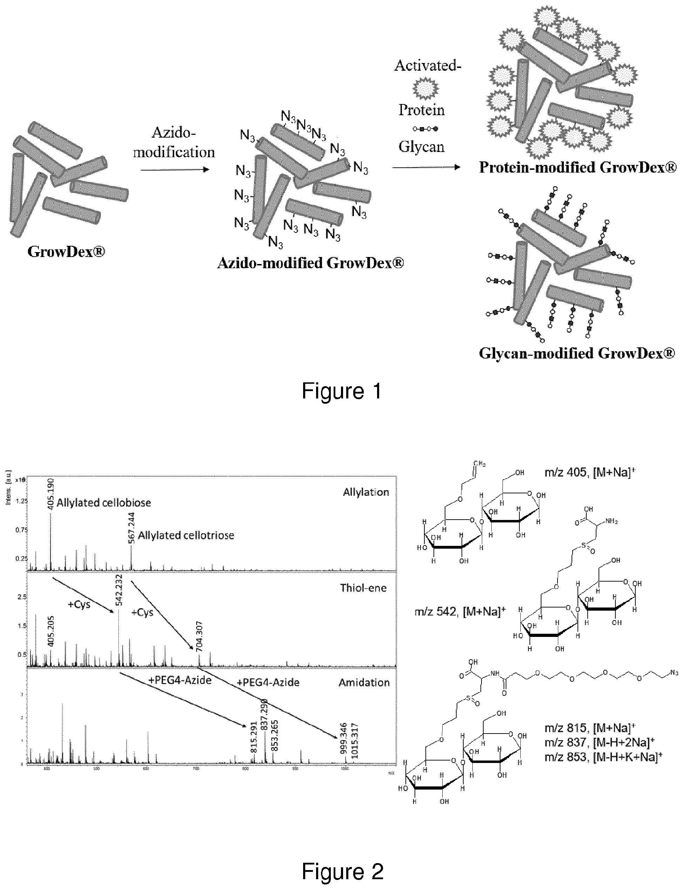

Figure 1 shows the generation of azido-modified and ligand-modified nanofibrillar cellulose schematically.

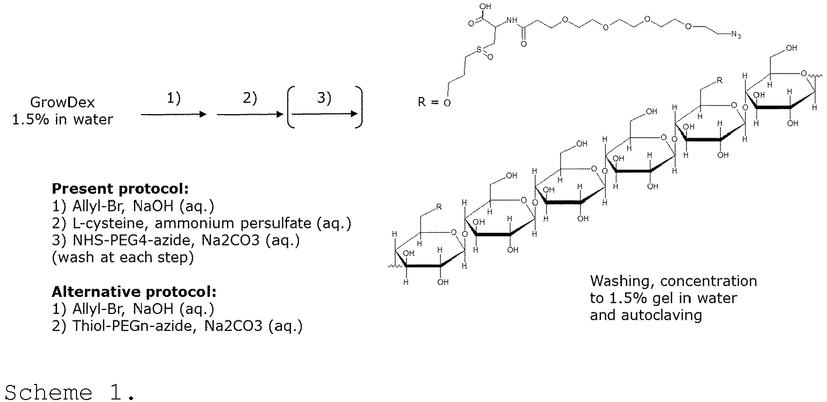

### Modification reactions

Synthetic route to the first product, azido-modified GrowDex, is shown in Scheme 1. Allylation was performed by reacting 0.75 % (w/v) GrowDex^{®} hydrogel with 0.62 % (v/v) allylbromide in 0.25 M sodium hydroxide at 60°C for 3 hours. The reaction was stopped by neutralization with acetic acid and washing with deionized water with centrifugation at 3000 rcf for 1 minute and removal of supernatant. The washing was repeated 5 times. Next, 0.75% (w/v) L-cysteine was added to 1 % (w/v) GrowDex^{®} hydrogel in 20 mM ammonium persulfate solution. After 3 hours at 50°C, the reaction was stopped by washing 5 times with deionized water as above. Finally, 0.75% (w/v) NHS-PEG4-azide reagent was added to 1 % (w/v) GrowDex^{®} hydrogel in 30 mM Na₂CO₃ buffer pH 9.3 solution. After 3 hours at RT, the reaction was stopped by washing 5 times with deionized water as above. Concentration to up to 1.5% was performed by centrifugation at 3000 rcf for a longer time and removal of supernatant.

### Autoclaving

The azido-modified material was autoclaved as about 1.5% solution without any visible changes in appearance.

### Characterization

After each reaction step of Scheme 1, an aliquot of the hydrogel was digested with cellulase (UPM Biochemicals) and analyzed by MALDI-TOF mass spectrometry, identifying the expected reaction products: 6-O-allyl, thiol-ene and azido-PEG4-amidated cellobiose and cellotriose (Figure 2). Figure 2 shows MALDI-TOF mass spectrometry of azido-modified GrowDex^{®}. Reaction products were analyzed after cellulase digestion. The expected modified di- and trisaccharides reaction products were observed.

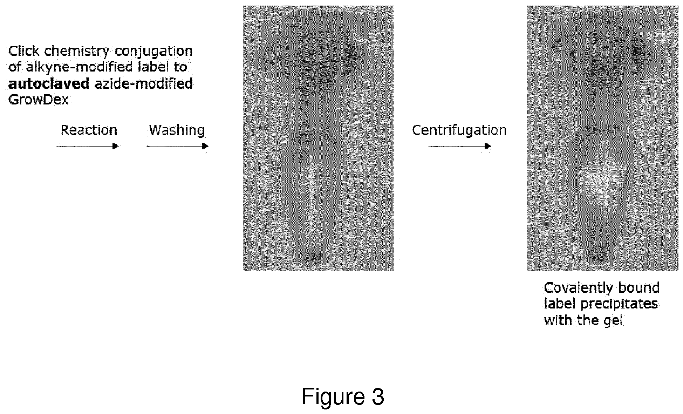

To verify that the azide functional groups had survived the autoclaving, their reactivity was verified by conjugation of a fluorescent label (Figure 3). Figure 3 shows that modified GrowDex^{®} is functional after autoclaving. Alkyne-functionalized fluorescent label (DBCO-Alexa) was reacted with autoclaved azido-modified GrowDex^{®}, after which the free unreacted label was washed away. The label precipitated together with the matrix during centrifugation, showing that it was covalently conjugated.

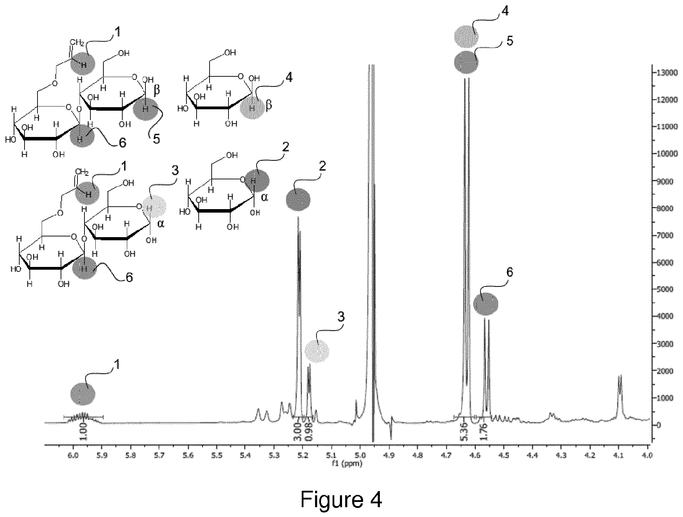

The allylated product was characterized by 1H-NMR spectroscopy after cellulase digestion to allow quantitation of allyl groups (Figure 4). Fig. 4 shows 1H-NMR spectroscopy of allylated GrowDex^{®}.Cellulase-digested allylated Growdex^{®} generated in reaction in 1 M NaOH; data not shown for 0.25 M NaOH solution (final optimized reaction condition). Quantitation of the integrals showed 11:1 relationship between glucose units and allyl groups in the sample. The reference numbers 1, 2, 3, 4, 5, and 6 in Fig. 4 indicate specific hydrogen atoms and peaks in the NMR spectrum corresponding to them.

### EXAMPLE 2 - Generation of a nanofibrillar cellulose hydrogel comprising protein-modified nanofibrillar cellulose

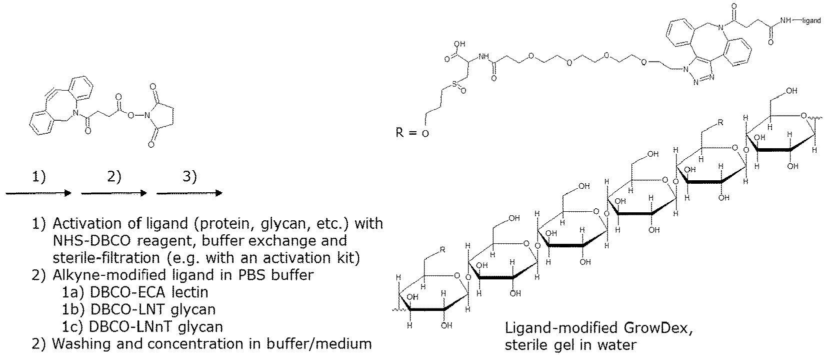

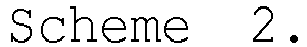

Synthesis route to Protein- and Glycan-modified GrowDex^{®}. Any water-soluble molecule that contains accessible amine groups can be activated with NHS-DBCO reagent and coupled to azido-modified GrowDex^{®} by click chemistry (copper-free azide-alkyne cycloaddition reaction). The reaction is essentially quantitative.

Synthetic route to the ligand-modified products, protein- and glycan-modified GrowDex^{®}, is shown in Scheme 2. The protein ligand in the present project was lectin from the plant *Erythrina cristagalli* (ECA), which binds to pluripotent stem cell (PSC) surfaces and promotes their adhesion to growth surface and efficient culturing in standard 2D cell culture (Mikkola et al. 2013, Stem Cells Dev. 22:707-16) . ECA (Sigma) was dissolved in phosphate-buffered saline (PBS) and alkyne-modified by adding 4:1 mol:mol NHS-DBCO reagent to ECA protein monomers and allowing to react in RT for 2 hours. Covalent conjugation of DBCO to ECA was verified by spectrophotometric analysis showing DBCO-derived absorbance signal at 309 nm after removal of free label by filtration (data not shown). The DBCO-ECA product was sterile-filtered and combined 100 µg/ml with 0.5% azido-modified GrowDex^{®}. After 2 hours of reaction in RT, the ECA-modified GrowDex^{®} was washed and transferred to iPS culture medium by centrifugation as above. Covalent conjugation of ECA to the cellulose matrix was verified by cellulase digestion of ECA-modified GrowDex^{®} followed by isolation of protein and MALDI-TOF mass spectrometry. ECA protein with the expected additional masses corresponding to cellulose fragment-linker additions were observed (data not shown).

### EXAMPLE 3 - Generation of a nanofibrillar cellulose hydrogel comprising glycan-modified nanofibrillar cellulose

The glycan ligands in the present project were the tetrasaccharides LNT (lacto-N-tetraose) and LNnT (lacto-N-neotetraose), which are components of PSC surface glycoconjugates and similarly as ECA, promote stem cell adhesion to growth surface and 2D cell culture (Mikkola et al., unpublished observations) . DBCO-LNT and DBCO-LNnT conjugates were synthesized at Glykos Finland Oy, purified with reversed-phase HPLC and characterized by MALDI-TOF mass spectrometry and 1H-NMR spectroscopy (data not shown). The DBCO-glycan products were sterile-filtered and combined with 0.5% azido-modified GrowDex^{®} as above.

### EXAMPLE 4 - Cell culture in nanofibrillar cellulose hydrogel comprising the modified nanofibrillar celluloses

### Cell culture

iPS cells were seeded in 0.5% hydrogel to a density of 1.0x105 cells/100µl hydrogel in Nutristem-medium (Stemgent) supplemented with 10 µM ROCK inhibitor (Y-27632 dihydrochloride, Calbiochem). Hydrogel with cells was aliquoted to 96-well plate and a 100 µl of Nutristem-medium supplemented with 10 µM ROCK inhibitor was added on top. Cells were cultured for 7-14 days with daily medium renewal.

### Cell counting assay

Cells were cultured for 8 days in GrowDex^{®} and modified GrowDex^{®} hydrogels, after which the hydrogels were degraded with 600 µg cellulase/mg cellulose overnight at +37°C. Cells were collected from 96-well plate into tubes and washed once with PBS. Spheroids were dissociated by treating with 0.5 mM EDTA (Invitrogen) for 2 minutes at +37°C and triturating with a pipette for 5-10 times. Cells were washed once with medium and counted. Three cell samples from every hydrogel condition were analyzed.

### Cell proliferation assay with PrestoBlue cell viability reagent

Cell viability was measured with resazurin-based PrestoBlue^{®} reagent from triplicate samples at time point 0 (immediately after seeding the cells in hydrogels) and at time point 7 (after 7 days of culture) . PrestoBlue^{®} reagent (Life Technologies) was added directly to the cell culture plate at 1:100 dilution and mixed. Cells were incubated at +37°C for 3.5 hours and absorbance was measured at 570 and 600 nm. Results were calculated according to the manufacturer's instructions. Briefly, the A600 was subtracted from A570 and the medium+hydrogel only control wells were averaged. The control average was subtracted from all sample wells and then the averages of triplicate samples were calculated.

### Cell proliferation assay with [2-¹⁴C] thymidine

Cell proliferation was analyzed by measuring incorporation of [2-¹⁴C] thymidine into cellular DNA and RNA. iPS cells were cultured for 5 days in 3D hydrogels, after which cells were cultured in the presence of 1.0 µCi/mL [2-¹⁴C] thymidine (Perkin Elmer) for 6, 24 and 48 hours. Cells were then washed twice with cold phosphate-buffered saline (PBS) to get rid of free [2-¹⁴C] thymidine. To solubilize cells, Solvable was added to samples and incubated at +60°C overnight. Before counting, scintillation fluid was added to the samples and the amount of incorporated [2-¹⁴C] thymidine was measured with a liquid scintillation counter (Wallac).

### Immunofluorescence staining

iPS cells were cultured for 10 days in GrowDex^{®} and modified GrowDex^{®} hydrogels with Nutristem-medium. Culture medium on top of hydrogel was removed (a 100 µl) and cells were fixed by adding a 100 µl of 8% paraformaldehyde and incubating for 90 minutes at room temperature (RT). Cells were then washed twice; PBS was added on top of hydrogel, incubated for 5 minutes and centrifuged at 200xg for 1 minute. Permeabilization of cells was done with 0.1% Triton X-100 in PBS overnight at +4°C (Phalloidin samples) or 2 hours at RT (TRA-1-60 and SSEA-4 -samples).

Cells were stained with anti-TRA-1-60 (R&D Systems) at 20 µg/ml and anti-SSEA-4 (Abcam) at 15 µg/ml concentration at +4°C overnight and washed twice with PBS as before. Secondary antibodies (DyLight488 anti-mouse IgM or AlexaFluor488 anti-mouse IgG) were added to a final concentration of 2 µg/ml and incubated at RT for 60 minutes. DAPI nuclear stain (Invitrogen) was added at the same time with secondary antibody to a final concentration of 2.5 µg/ml.

Cells were stained with Alexa Fluor 488 phalloidin (Life Technologies) at 1:400 dilution at RT for 60 minutes. DAPI nuclear stain (Invitrogen) was added at the same time with phalloidin to a final concentration of 2.5 µg/ml. Cells were washed twice with PBS as before and finally transferred to a black 96-well plate for confocal imaging and to microscope slides for fluorescence microscope imaging.

In confocal microscopy, the spheroids were photographed with 31 Marianas (31 intelligent Imaging Innovations) fluorescence microscope equipped with spinning disk confocal, 20x/0.4 LD Plan-Neofluar Ph2 Corr WD=7.9 M27 objective, violet (solid state 405nm/100mW) and blue (solid state 488nm/50mW) lasers and Zeiss Axio Observer Z1 inverted microscope.

Alternatively, the spheroids were observed and photographed with Zeiss Axio Scope A1 equipped with ProgRes C5 CCD camera (JENOPTIK) and 10x or 20x objectives (N-ACHROPLAN 10x/0.25 Ph1, Plan-Neofluar 20x/0.50 Ph2) using appropriate filter sets.

### Growth characteristics

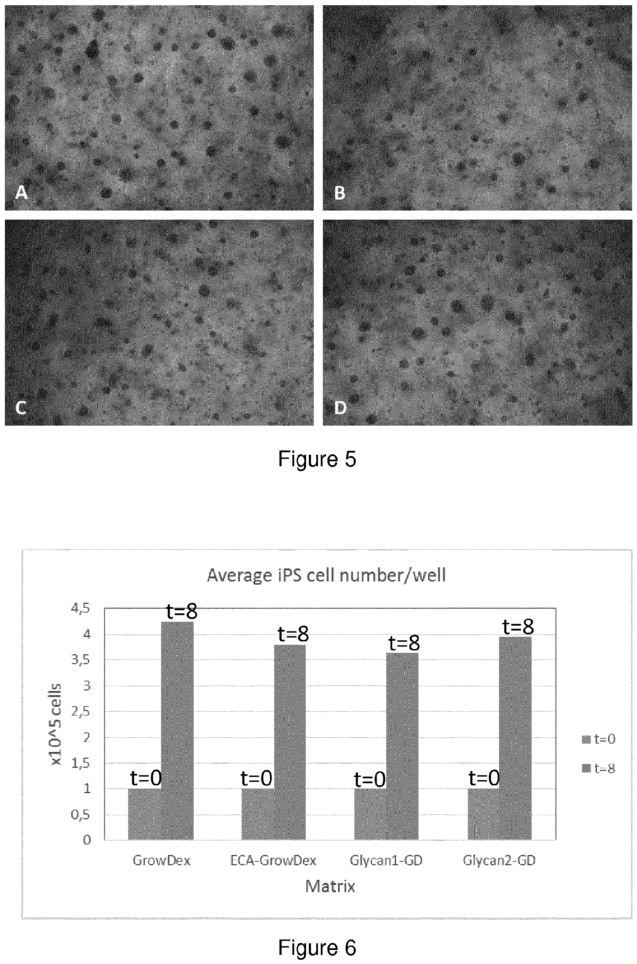

After 9 days of culture in GrowDex^{®} and modified GrowDex^{®} hydrogels, iPS cells had formed small spheroids (Figure 5). Fig. 5 illustrates iPS cells in the different 3D hydrogels after 9 days of culture. **A)** GrowDex^{®}, **B)** ECA-GrowDex^{®}, **C)** LNT-GrowDex^{®} and **D)** LNnT-GrowDex^{®}. Spheroid size was not significantly different between the samples. Cell proliferation in 3D hydrogel culture was measured with three different methods. First, a crude cell number assay was performed by counting the cells after 8 days of culture, degradation of hydrogel and dissociation of spheroids. No difference between GrowDex^{®} and modified GrowDex^{®} hydrogels was observed when comparing the cell numbers (Figure 6) . Fig. 6 shows cell counts of iPS cells in different 3D hydrogels. Total number of cells in 3D hydrogel cultures after 8 days, averaged from triplicate samples.

The counts are total cell number including also dead cells (cell viability was not determined).

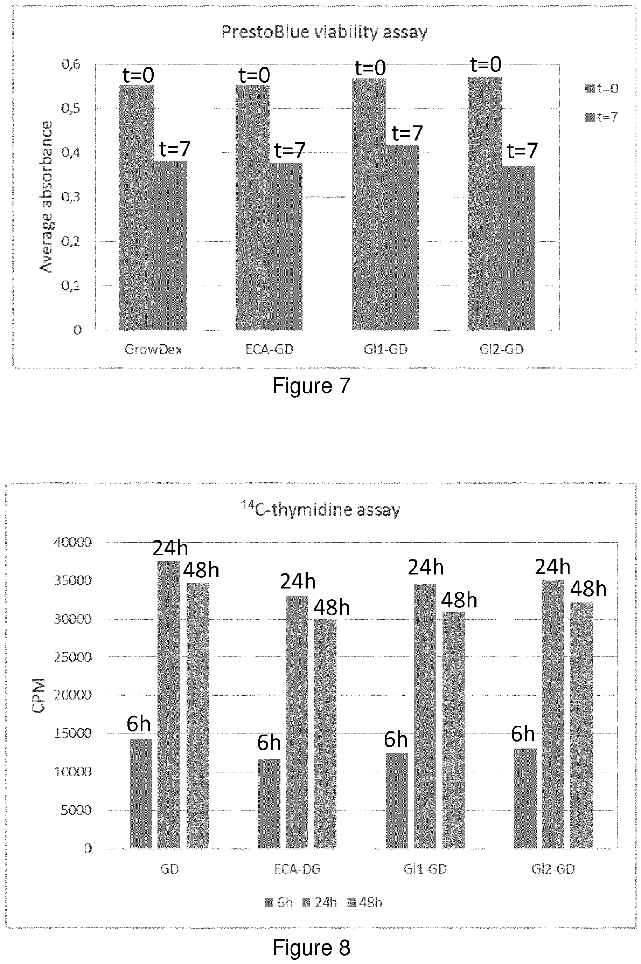

Cell viability and proliferation was also determined with PrestoBlue^{®} cell viability assay. Viability was determined directly after seeding the cells in hydrogels and again after 7 days of culture. The viability was diminished during culture when the results of day 0 and day 7 were compared (Figure 7). Fig. 7 demonstrates cell viability of iPS cells in different 3D hydrogels at time points 0 (day 0) and 7 (day 7). No difference in the viability between GrowDex^{®} and modified GrowDex^{®} hydrogels was observed. Incorporation of [2-¹⁴C] thymidine into cellular nucleic acids was used to compare the proliferation rate of iPS cells in GrowDex^{®} and modified GrowDex^{®} hydrogels. After 5 days of culture the cells were incubated with [2-¹⁴C] thymidine for 6, 24 and 48 hours, after which the radioactivity in cellular nucleic acids was measured with a scintillation counter. iPS cells grown in modified GrowDex^{®} hydrogel samples did not incorporate higher amounts of [2-¹⁴C] thymidine in any time points compared to GrowDex^{®} (Figure 8), which is compatible with the results from the other proliferation assays. Fig. 8 shows the results of the cell proliferation assay. iPS cell proliferation was assayed with [¹⁴C]thymidine incorporation. Cell proliferation in modified GrowDex^{®} hydrogels was not higher than with original GrowDex^{®}. n=2 at each time point.

### Stem cell characteristics - Anti-TRA-1-60 staining

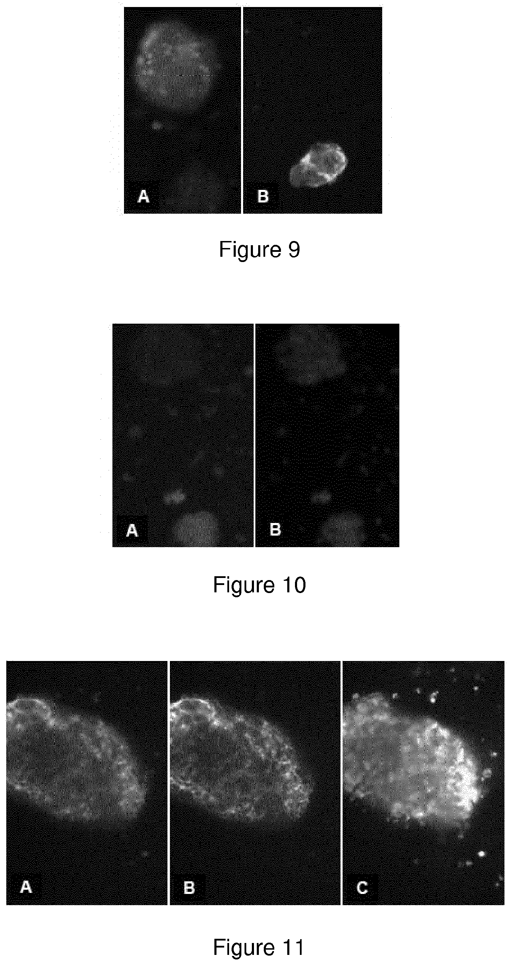

Some small spheroids showed overall Tra-1-60 staining, but mostly staining was confined to subset of cells within the spheroids (Figure 9). Fig. 9 shows anti-Tra-1-60 staining in spheroids grown in modified GrowDex^{®}. **A)** Scattered Tra-1-60 positive cells (green) are seen in the upper spheroid whereas no or very low levels of Tra-1-60 positive cells are seen in the lower spheroid grown in ECA-GrowDex^{®} (spheroid diameter about 100 µm). **B)** A confocal image of a spheroid shows many Tra-1-60 positive cells (LNnT-GrowDex^{®}, spheroid length about 70 µm). Blue DAPI stains shows nuclei. Original magnifications 20x.

By counting random spheroids (at 10x magnification) and their Tra-1-60 staining, about 59%, 52%, 64%, and 64% of unmodified GrowDex^{®}, ECA-, LNT- and LNnT-modified gels, respectively, showed Tra-1-60 staining (of 20-30 randomly selected spheroids). Typically, the largest spheroids did not show Tra-1-60 positive cells, possibly indicating start of differentiation.

### Stem cell characteristics - Anti-SSEA-4 staining

In general, SSEA-4 staining was mostly confined to small spheroids and usually the staining appeared in all or almost all cells of the spheroid **(****Figure 10).** Fig. 10 shows anti-SSEA-4 staining in spheroids grown in ECA-GrowDex^{®}. **A)** The lower spheroid shows many SSEA-4 positive cells whereas no or very low levels of SSEA-4 positive cells are seen in the upper spheroid. **B)** Nuclei are stained blue (DAPI). Original magnification 20x, spheroid diameters about 80 and 50 µm.

In some spheroids, a subset of cells showed SSEA-4 staining. By counting random spheroids (at 10x magnification) and their SSEA-4 staining, about 30%, 45%, 70%, and 50% of untreated GrowDex^{®}, ECA-, LNT- and LNnT-modified gels, respectively, showed SSEA-4 staining (of 20-30 randomly selected spheroids). In general, intensity of anti-SSEA-4 staining was weaker compared to that of anti-Tra-1-60 staining.

### General characteristics

Phalloidin-Alexa488 staining was used to depict overall cellular morphology (phalloidin binds to actin). No gross changes were detected between the spheroids cultured in the unmodified or modified GrowDex^{®}. The majority of spheroids were in range of small to medium but all gels included also "large" sized spheroids and their number was approximately the same (20, 9, 16 and 10 large spheroids in unmodified, ECA-, LNT- and LNnT-GrowDex^{®}, respectively, when counted in a 96-well).

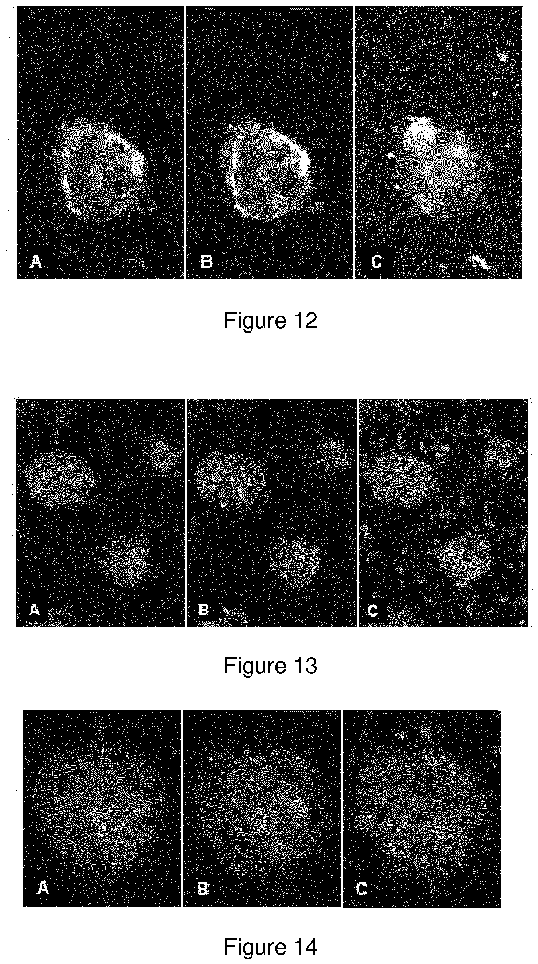

Figures 11-14 show Alexa488-phalloidin stainings of the spheroids. Fig. 11 shows Alexa488-phalloidin staining in a "large" spheroid grown in ECA-GrowDex^{®}, a confocal image. **A)** Composite image of the spheroid, green shows A488-phalloidin and blue shows nuclei. **B)** Green channel showing A488-phalloidin staining. **C)** Blue channel showing DAPI staining. The spheroid length is approx. 220 µm (original magnification 20x). Fig. 12 illustrates Alexa488-phalloidin staining in a spheroid grown in glycan1-GrowDex^{®}, a confocal image. **A)** Composite image of the spheroid, green shows A488-phalloidin and blue shows nuclei. **B)** Green channel showing A488-phalloidin staining. **C)** Blue channel showing DAPI staining. Single DAPI positive fragments are seen outside the spheroid but these do not show A488-phalloidin staining. The spheroid diameter about 70 µm (original magnification 20x). Fig. 13 shows Alexa488-phalloidin staining in a spheroid grown in unmodified GrowDex^{®}. **A)** Composite image of the spheroid, green shows A488-phalloidin and blue shows nuclei. **B)** Green channel showing A488-phalloidin staining. **C)** Blue channel showing DAPI staining. Single DAPI positive fragments or fragment clusters are seen outside the spheroid and these do not show A488-phalloidin staining. Original magnification 20x. Fig. 14 shows Alexa488-phalloidin staining in a spheroid grown in unmodified GrowDex^{®}. **A)** Composite image of the spheroid, green shows A488-phalloidin and blue shows nuclei. **B)** Green channel showing A488-phalloidin staining. **C)** Blue channel showing DAPI staining. DAPI positive fragment clusters are seen in the spheroid. Original magnification 20x.

Many single cells appeared throughout the all the gels exhibiting Tra-1-60, SSEA-4 or phalloidin staining. However, majority of DAPI-stained structures were most likely fragmented nuclei of dead or cells undergoing apoptosis (as evidenced by DAPI-positive staining but not A488-phalloidin staining).

Some spheroids also contained fragmented nuclei (without surrounding phalloidin staining) resulting either from normal cellular processes or from growth conditions not optimal for the spheroids (apoptosis). The fragmented nuclei were seen in spheroids of all gels and no effort was taken to quantify proportions of fragmented nuclei in spheroids or outside spheroids. The single cells are also counted in cellular assays.

### EXAMPLE 4 - Rheological measurements of the nanofibrillar cellulose hydrogels

Three samples were prepared for viscosimetric analyses:
1. 0.5% GrowDex^{®} in water, 2 ml
2. 0.5% azido-modified GrowDex^{®} in water, 2 ml
3. 0.5% ECA-modified GrowDex^{®} in water, 2 ml

GrowDex^{®} concentration in each sample was analyzed by colorimetric resorcinol assay, which correlates with the glucose monomer concentration. All samples were diluted to 0.5 % (w/v) concentration by comparison to 0.5 % (w/v) GrowDex^{®} preparate.

To verify the success of modification, rheological measurements of the samples in the form of nanofibrillar cellulose hydrogels were carried out with a stress controlled rotational rheometer (ARG2, TA instruments, UK) equipped with 20 mm plate geometry. The stress sweep measurements of unmodified and modified nanofibrillar cellulose hydrogels were performed in 0.5 wt% to verify that the gel strength does not change due to modification. The stress sweep was measured in a shear stress range of 0.01 - 100 Pa at the frequency 0.1 Hz, at 22 °C.

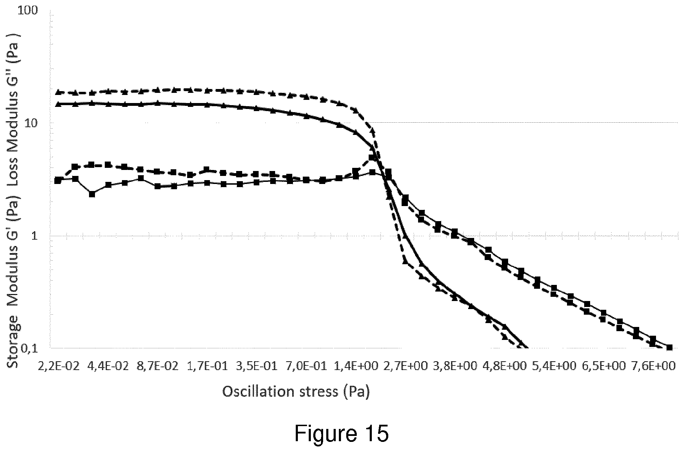

Figure 15 illustrates the visco-elastic properties of 0.5 % nanocellulose dispersions of unmodified sample (solid line) and modified sample (dotted line) by stress-sweep measurement. Stress dependence of G' (the storage modulus, Δ) and G" (the loss modulus, □) are presented.

Samples 1. and 2. (GrowDex^{®} and azido-modified GrowDex^{®}) had the same level of viscosity, demonstrating that the azido-modification had no effect on viscosity. Sample 3. (ECA-modified GrowDex^{®}) had a somewhat lower viscosity but was in the form of a gel.

### EXAMPLE 5 - Kit for preparing the ligand-modified nanofibrillar cellulose hydrogel

Contents of the ligand conjugation kit:
- DBCO: 0.5 mg DBCO-sulfo-NHS ester dried to bottom of tube
- PBS: 1 ml sterile phosphate-buffered saline (PBS)
- Amicon: 10 kDa MWCO centrifugal filter
- nanofibrillar cellulose-Azide: 2.5 ml azido-modified nanofibrillar cellulose matrix, 1.5% sterile gel in water

Instructions for the use of the kit:
1. Dissolve calculated amount of protein/ligand in buffer.
2. Add the calculated amount of DBCO solution to the ligand solution and mix.
3. Incubate at room temperature.
4. Transfer the DBCO-ligand solution into the centrifugal filter tube to remove excess unreacted DBCO by repeated centrifuging and addition of buffer.
5. Add DBCO-ligand reagent to nanofibrillar cellulose-Azide matrix and mix thoroughly to distribute the ligand evenly in the matrix.
6. Incubate at room temperature.
7. Change the ligand-modified nanofibrillar cellulose-Azide matrix into an appropriate culture medium.

It is obvious to a person skilled in the art that with the advancement of technology, the basic idea may be implemented in various ways. The embodiments are thus not limited to the examples described above; instead they may vary within the scope of the claims.

The embodiments described hereinbefore may be used in any combination with each other. Several of the embodiments may be combined together to form a further embodiment. A method, a product, a system, or a use, disclosed herein, may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" is used in this specification to mean including the feature(s) or act(s) followed thereafter, without excluding the presence of one or more additional features or acts.

## Claims

1. A nanofibrillar cellulose hydrogel comprising azido-modified nanofibrillar cellulose having a substituent represented by the formula -O-(CH₂)ₙ-S(O)ₘ-L₁-N₃, wherein n is in the range of 1 to 10; m is 0 or 1; and L₁ is a linker; wherein the substituent is attached to a carbon of one or more glucosyl units of the azido-modified nanofibrillar cellulose, thus forming an ether bond to the carbon.

2. The nanofibrillar cellulose hydrogel according to claim 1, wherein L₁ represents -CH₂-CH₂(R₁)-NH-L₂-, wherein R₁ is absent or -COOH, and L₂ is a linker.

3. The nanofibrillar cellulose hydrogel according to claim 2, wherein L₂ represents C(O)-(CH₂-CH₂-O)ₒ-CH₂-CH₂-, wherein o is 0 or greater.

4. The nanofibrillar cellulose hydrogel according to any one of claims 1 - 3, wherein at least one or more of the one or more glucosyl units are β1,4-D-glucopyranosyl units, and the carbon of the one of more β1,4-D-glucopyranosyl units to which the substituent is attached is carbon 6.

5. The nanofibrillar cellulose hydrogel according to any one of claims 1 - 4, wherein the azido-modified nanofibrillar cellulose has a degree of substitution of at least about 0.0001, or at least about 0.01.

6. A kit or a solid support comprising the nanofibrillar cellulose hydrogel according to any one of claims 1 - 5 and optionally a reaction buffer and/or instructions for use, wherein the solid support has a recess for receiving or containing the nanofibrillar cellulose hydrogel.

7. The kit or solid support according to claim 6, wherein the kit or solid support further comprises a ligand having a cyclic or acyclic alkyne group, or a linker compound conjugable to a ligand and having a cyclic or acyclic alkyne group.

8. A method for preparing the nanofibrillar cellulose hydrogel according to any one of claims 1 - 5, wherein the method comprises
alkenylating a hydroxyl group of one or more glucosyl units of nanofibrillar cellulose with an alkenylating agent to obtain alkenylated nanofibrillar cellulose, and
conjugating an azide-containing compound with the alkenylated nanofibrillar cellulose, thereby obtaining the nanofibrillar cellulose hydrogel comprising the azido-modified nanofibrillar cellulose;
wherein the alkenylating agent has a structure represented by the formula X-(CH₂)ₙCH=CH₂, wherein n is in the range from 1 to 8, and X is Br, Cl, or I.

9. The method according to claim 8, wherein the method comprises reacting a thiol group-containing compound with the alkenylated nanofibrillar cellulose, wherein the thiol group-containing compound further has an azide group, thereby obtaining the nanofibrillar cellulose hydrogel comprising the azido-modified nanofibrillar cellulose; or
wherein the method comprises reacting a thiol group-containing compound with the alkenylated nanofibrillar cellulose, wherein the thiol group-containing compound further has an amino group, thereby obtaining an amino-modified nanofibrillar cellulose, and reacting a compound having a functional group capable of reacting with the amino group with the amino-modified nanofibrillar cellulose, wherein the compound having the functional group further has an azide group, thereby obtaining the nanofibrillar cellulose hydrogel comprising the azido-modified nanofibrillar cellulose.

10. The method according to claim 9, wherein the thiol group-containing compound is cysteine, cysteamine or a combination or a mixture thereof.

11. A nanofibrillar cellulose hydrogel comprising ligand-modified nanofibrillar cellulose, wherein the ligand-modified nanofibrillar cellulose has one or more ligands covalently bound thereto, and the one or more ligands comprise at least one of a protein, a peptide, a glycan or a nanofibrillar cellulose molecule; wherein the ligand-modified nanofibrillar cellulose has a substituent represented by the formula -O-(CH₂)ₙ-S(O)ₘ-L₁-D, wherein n is in the range of 1 to 10; m is 0 or 1; L₁ is a linker; and D represents the ligand covalently bound to the linker and a triazole group formed by a reaction between an azido group of the azido-modified nanofibrillar cellulose of the nanofibrillar cellulose hydrogel according to any one of claims 1 - 5 and a cyclic or acyclic alkyne group of the ligand, wherein the triazole group is optionally a 1,5-disubstituted 1,2,3-triazole; and wherein the substituent is attached to a carbon of one or more glucosyl units of the ligand-modified nanofibrillar cellulose, thus forming an ether bond to the carbon.

12. The nanofibrillar cellulose hydrogel according to claim 11, wherein D represents the ligand covalently bound to the linker and the triazole group formed by a reaction between the azide group of the nanofibrillar cellulose of the nanofibrillar cellulose hydrogel according to any one of claims 1 - 5 and the cyclic or acyclic alkyne group of the ligand, so that the ligand is covalently bound to the linker via the triazole group.

13. The nanofibrillar cellulose hydrogel according to claim 11, wherein L₁ represents -CH₂-CH₂(R₁)-NH-L₂-, wherein R₁ is absent or -COOH, and L₂ is a linker.

14. A method for preparing a nanofibrillar cellulose hydrogel according to any one of claims 11 - 13, the method comprising contacting the nanofibrillar cellulose hydrogel according to any one of claims 1 - 5 with a ligand having a cyclic or acyclic alkyne group.

15. The method according to claim 14, wherein the method comprises conjugating a linker compound having a cyclic or acyclic alkyne group to the ligand, thereby obtaining the ligand having the cyclic or acyclic alkyne group.

16. The kit or solid support according to claim 7, the nanofibrillar cellulose hydrogel according to any one of claims 11 - 13, or the method according to claim 14 or 15, wherein the cyclic alkyne group is DBCO, OCT, MOFO, DIFO, DIFO2, DIFO3, DIMAC, DIBO, BARAC, BCN, Sondheimer diyne, TMDIBO, S-DIBO, COMBO, or PYRROC.

17. Use of the azido-modified nanofibrillar cellulose hydrogel according to any one of claims 1 - 5 or of the ligand-modified nanofibrillar cellulose hydrogel according to any one of claims 11 - 13 for maintaining, transporting, isolating, culturing, propagating, passaging, differentiating or transplanting of cells or tissues.

## Patentansprüche

1. Nanofibrilläres Zellulose-Hydrogel, umfassend azido-modifizierte nanofibrilläre Zellulose mit einem Substituenten, repräsentiert durch die Formel -O-(CH₂)ₙ-S(O)ₘ-L₁-N₃, wobei n im Bereich von 1 bis 10 liegt; m 0 oder 1 ist; und L₁ ein Linker ist; wobei der Substituent an einen Kohlenstoff einer oder mehrerer Glucosyleinheiten der azido-modifizierten nanofibrillären Zellulose angeheftet ist, wodurch eine Etherbindung an den Kohlenstoff gebildet wird.

2. Nanofibrilläres Zellulose-Hydrogel gemäß Anspruch 1, wobei L₁ -CH₂-CH₂(R₁)-NH-L₂- repräsentiert, wobei R₁ fehlt oder -COOH ist, und L₂ ein Linker ist.

3. Nanofibrilläres Zellulose-Hydrogel gemäß Anspruch 2, wobei L₂ C(O)-(CH₂-CH₂-O)ₒ-CH₂-CH₂- repräsentiert, wobei o 0 oder größer ist.

4. Nanofibrilläres Zellulose-Hydrogel gemäß irgendeinem der Ansprüche 1-3, wobei wenigstens eine oder mehrere der einen oder mehreren Glucosyleinheiten β1,4-D-Glucopyranosyleinheiten sind und der Kohlenstoff der einen oder mehreren β1,4-D-Glucopyranosyleinheiten, an die der Substituent angeheftet ist, Kohlenstoff 6 ist.

5. Nanofibrilläres Zellulose-Hydrogel gemäß irgendeinem der Ansprüche 1-4, wobei die azido-modifizierte nanofibrilläre Zellulose einen Substitutionsgrad von wenigstens etwa 0,0001 oder wenigstens etwa 0,01 hat.

6. Kit oder feste Basis, umfassend das nanofibrilläre Zellulose-Hydrogel gemäß irgendeinem der Ansprüche 1-5 und optional einen Reaktionspuffer und/oder Anweisungen zur Verwendung, wobei die feste Basis eine Aussparung aufweist, um das nanofibrilläre Zellulose-Hydrogel aufzunehmen oder zu enthalten.

7. Kit oder feste Basis gemäß Anspruch 6, wobei das Kit oder die feste Basis weiterhin einen Liganden mit einer zyklischen oder azyklischen Alkyngruppe oder eine Linkerverbindung, die mit einem Liganden konjugierbar ist und eine zyklische oder azyklische Alkyngruppe aufweist, umfasst.

8. Verfahren zum Herstellen des nanofibrillären Zellulose-Hydrogels gemäß irgendeinem der Ansprüche 1-5, wobei das Verfahren umfasst
Alkenylieren einer Hydroxylgruppe einer oder mehrerer Glucosyleinheiten von nanofibrillärer Zellulose mit einem Alkenylierungsagens, um alkenylierte nanofibrilläre Zellulose zu erhalten, und
Konjugieren einer Azid-enthaltenden Verbindung mit der alkenylierten nanofibrillären Zellulose, dadurch Erhalten des nanofibrillären Zellulose-Hydrogels, welches die azido-modifizierte nanofibrilläre Zellulose umfasst;
wobei das Alkenylierungsagens eine Struktur aufweist, repräsentiert durch die Formel X-(CH₂)ₙCH=CH₂, wobei n im Bereich von 1 bis 8 liegt und X Br, Cl oder I ist.

9. Verfahren gemäß Anspruch 8, wobei das Verfahren das Umsetzen einer Thiolgruppe-enthaltenden Verbindung mit der alkenylierten nanofibrillären Zellulose umfasst, wobei die Thiolgruppe-enthaltende Verbindung weiterhin eine Azidgruppe aufweist, dadurch Erhalten des nanofibrillären Zellulose-Hydrogels, welches die azido-modifizierte nanofibrilläre Zellulose umfasst; oder wobei das Verfahren das Umsetzen einer Thiolgruppe-enthaltenden Verbindung mit der alkenylierten nanofibrillären Zellulose umfasst, wobei die Thiolgruppe-enthaltende Verbindung weiterhin eine Aminogruppe aufweist, dadurch Erhalten einer amino-modifizierten nanofibrillären Zellulose, und Umsetzen einer Verbindung mit einer funktionellen Gruppe, die zur Reaktion mit der Aminogruppe in der Lage ist, mit der amino-modifizierten nanofibrillären Zellulose, wobei die Verbindung mit der funktionellen Gruppe weiterhin eine Azidgruppe aufweist, dadurch Erhalten des nanofibrillären Zellulose-Hydrogels, welches die azido-modifizierte nanofibrilläre Zellulose umfasst.

10. Verfahren gemäß Anspruch 9, wobei die Thiolgruppe-enthaltende Verbindung Cystein, Cysteamin oder eine Kombination oder ein Gemisch davon ist.

11. Nanofibrilläres Zellulose-Hydrogel, umfassend Liganden-modifizierte nanofibrilläre Zellulose, wobei die Liganden-modifizierte nanofibrilläre Zellulose einen oder mehrere kovalent daran gebundene Liganden aufweist und der eine oder die mehreren Liganden wenigstens eines von einem Protein, einem Peptid, einem Glycan oder einem nanofibrillären Zellulosemolekül umfassen; wobei die Liganden-modifizierte nanofibrilläre Zellulose einen Substituenten aufweist, repräsentiert durch die Formel -O-(CH₂)ₙ-S(O)ₘ-L₁-D, wobei n im Bereich von 1 bis 10 liegt; m 0 oder 1 ist; L₁ ein Linker ist; und D den kovalent an den Linker gebundenen Liganden und eine Triazolgruppe, gebildet durch eine Reaktion zwischen einer Azidogruppe der azido-modifizierten nanofibrillären Zellulose des nanofibrillären Zellulose-Hydrogels gemäß irgendeinem der Ansprüche 1-5 und einer zyklischen oder azyklischen Alkyngruppe des Liganden, repräsentiert, wobei die Triazolgruppe optional ein 1,5-disubstituiertes 1,2,3-Triazol ist; und wobei der Substituent an einen Kohlenstoff einer oder mehrerer Glucosyleinheiten der Liganden-modifizierten nanofibrillären Zellulose angeheftet ist, wodurch eine Etherbindung an den Kohlenstoff gebildet wird.

12. Nanofibrilläres Zellulose-Hydrogel gemäß Anspruch 11, wobei D den kovalent an den Linker gebundenen Liganden und die Triazolgruppe, gebildet durch eine Reaktion zwischen der Azidgruppe der nanofibrillären Zellulose des nanofibrillären Zellulose-Hydrogels gemäß irgendeinem der Ansprüche 1-5 und der zyklischen oder azyklischen Alkyngruppe des Liganden, repräsentiert, so dass der Ligand über die Triazolgruppe kovalent an den Linker gebunden ist.

13. Nanofibrilläres Zellulose-Hydrogel gemäß Anspruch 11, wobei L₁ -CH₂-CH₂(R₁)-NH-L₂- repräsentiert, wobei R₁ fehlt oder -COOH ist und L₂ ein Linker ist.

14. Verfahren zum Herstellen eines nanofibrillären Zellulose-Hydrogels gemäß irgendeinem der Ansprüche 11-13, wobei das Verfahren das Inkontaktbringen des nanofibrillären Zellulose-Hydrogels gemäß irgendeinem der Ansprüche 1-5 mit einem Liganden mit einer zyklischen oder azyklischen Alkyngruppe umfasst.

15. Verfahren gemäß Anspruch 14, wobei das Verfahren das Konjugieren einer Linkerverbindung mit einer zyklischen oder azyklischen Alkyngruppe an den Liganden umfasst, dadurch Erhalten des Liganden mit der zyklischen oder azyklischen Alkyngruppe.

16. Kit oder feste Basis gemäß Anspruch 7, nanofibrilläres Zellulose-Hydrogel gemäß irgendeinem der Ansprüche 11-13 oder Verfahren gemäß Anspruch 14 oder 15, wobei die zyklische Alkyngruppe DBCO, OCT, MOFO, DIFO, DIFO2, DIFO3, DIMAC, DIBO, BARAC, BCN, Sondheimer-Diyn, TMDIBO, S-DIBO, COMBO oder PYRROC ist.

17. Verwendung des azido-modifizierten nanofibrillären Zellulose-Hydrogels gemäß irgendeinem der Ansprüche 1-5 oder des Liganden-modifizierten nanofibrillären Zellulose-Hydrogels gemäß irgendeinem der Ansprüche 11-13 zum Aufrechterhalten, Transportieren, Isolieren, Kultivieren, Vermehren, Passagieren, Differenzieren oder Transplantieren von Zellen oder Geweben.

## Revendications

1. Hydrogel de cellulose nanofibrillaire comprenant une cellulose nanofibrillaire à modification azido ayant un substituant représenté par la formule -O-(CH₂)ₙ-S(O)ₘ-L₁-N₃, où n est situé dans la plage allant de 1 à 10 ; m vaut 0 ou 1 ; et L₁ est un lieur ; dans lequel le substituant est rattaché à un carbone d'un ou plusieurs motifs glucosyle de la cellulose nanofibrillaire à modification azido, en formant ainsi une liaison éther au carbone.

2. Hydrogel de cellulose nanofibrillaire selon la revendication 1, dans lequel L₁ représente -CH₂-CH₂(R₁)-NH-L₂-, où R₁ est -COOH ou est absent, et L₂ est un lieur.

3. Hydrogel de cellulose nanofibrillaire selon la revendication 2, dans lequel L₂ représente C(O)-(CH₂-CH₂-O)ₒ-CH₂-CH₂-, où o vaut 0 ou plus.

4. Hydrogel de cellulose nanofibrillaire selon l'une quelconque des revendications 1 à 3, dans lequel au moins un ou plusieurs des un ou plusieurs motifs glucosyle sont des motifs β1,4-D-glucopyranosyle, et le carbone des un ou plusieurs motifs β1,4-D-glucopyranosyle auquel le substituant est rattaché est le carbone 6.

5. Hydrogel de cellulose nanofibrillaire selon l'une quelconque des revendications 1 à 4, dans lequel la cellulose nanofibrillaire à modification azido a un degré de substitution d'au moins environ 0,0001, ou d'au moins environ 0,01.

6. Trousse ou support solide comprenant l'hydrogel de cellulose nanofibrillaire selon l'une quelconque des revendications 1 à 5 et éventuellement un tampon réactionnel et/ou des instructions d'utilisation, où le support solide a un évidement pour recevoir ou contenir l'hydrogel de cellulose nanofibrillaire.

7. Trousse ou support solide selon la revendication 6, où la trousse ou le support solide comprend en outre un ligand ayant un groupe alcyne cyclique ou acyclique, ou un composé lieur conjugable à un ligand et ayant un groupe alcyne cyclique ou acyclique.

8. Procédé pour préparer l'hydrogel de cellulose nanofibrillaire selon l'une quelconque des revendications 1 à 5, où le procédé comprend les étapes consistant en
l'alcénylation d'un groupe hydroxyle d'un ou plusieurs motifs glucosyle de cellulose nanofibrillaire avec un agent d'alcénylation pour que soit obtenue une cellulose nanofibrillaire alcénylée, et
la conjugaison d'un composé contenant un azoture avec la cellulose nanofibrillaire alcénylée, ce qui donne ainsi l'hydrogel de cellulose nanofibrillaire comprenant la cellulose nanofibrillaire à modification azido ;
dans lequel l'agent d'alcénylation a une structure représentée par la formule X-(CH₂)ₙCH=CH₂ dans laquelle n est situé dans la plage allant de 1 à 8 et X est Br, Cl ou I.

9. Procédé selon la revendication 8, où le procédé comprend la réaction d'un composé contenant un groupe thiol avec la cellulose nanofibrillaire alcénylée, où le composé contenant un groupe thiol a en outre un groupe azoture, ce qui donne ainsi l'hydrogel de cellulose nanofibrillaire comprenant la cellulose nanofibrillaire à modification azido ; ou
où le procédé comprend la réaction d'un composé contenant un groupe thiol avec la cellulose nanofibrillaire alcénylée, où le composé contenant un groupe thiol a en outre un groupe amino, ce qui donne ainsi une cellulose nanofibrillaire à modification amino, et la réaction d'un composé ayant un groupe fonctionnel capable de réagir avec le groupe amino avec la cellulose nanofibrillaire à modification amino, où le composé ayant le groupe fonctionnel a en outre un groupe azoture, ce qui donne ainsi l'hydrogel de cellulose nanofibrillaire comprenant la cellulose nanofibrillaire à modification azido.

10. Procédé selon la revendication 9, dans lequel le composé contenant un groupe thiol est la cystéine, la cystéamine ou une combinaison ou un mélange de celles-ci.

11. Hydrogel de cellulose nanofibrillaire comprenant une cellulose nanofibrillaire modifiée par un ligand, dans lequel la cellulose nanofibrillaire modifiée par un ligand a un ou plusieurs ligands liés de manière covalente à celle-ci, et les un ou plusieurs ligands comprennent au moins l'un parmi une protéine, un peptide, un glycane et une molécule de cellulose nanofibrillaire ; dans lequel la cellulose nanofibrillaire modifiée par un ligand a un substituant représenté par la formule -O- (CH₂)ₙ-S(O)ₘ-L₁-D, dans laquelle n est situé dans la plage allant de 1 à 10 ; m vaut 0 ou 1 ; L₁ est un lieur ; et D représente le ligand lié de manière covalente au lieur et à un groupe triazole formé par une réaction entre un groupe azido de la cellulose nanofibrillaire à modification azido de l'hydrogel de cellulose nanofibrillaire selon l'une quelconque des revendications 1 à 5 et un groupe alcyne cyclique ou acyclique du ligand, où le groupe triazole est éventuellement un 1,2,3-triazole 1,5-disubstitué ; et où le substituant est rattaché à un carbone d'un ou plusieurs motifs glucosyle de la cellulose nanofibrillaire modifiée par un ligand, en formant ainsi une liaison éther au carbone.

12. Hydrogel de cellulose nanofibrillaire selon la revendication 11, dans lequel D représente le ligand lié de manière covalente au lieur et au groupe triazole formé par une réaction entre un groupe azido de la cellulose nanofibrillaire de l'hydrogel de cellulose nanofibrillaire selon l'une quelconque des revendications 1 à 5 et le groupe alcyne cyclique ou acyclique du ligand, si bien que le ligand est lié de manière covalente au lieur via le groupe triazole.

13. Hydrogel de cellulose nanofibrillaire selon la revendication 11, dans lequel L₁ représente -CH₂-CH₂(R₁)-NH-L₂-, où R₁ est -COOH ou est absent, et L₂ est un lieur.

14. Procédé pour préparer un hydrogel de cellulose nanofibrillaire selon l'une quelconque des revendications 11 à 13, le procédé comprenant l'étape de mise en contact de l'hydrogel de cellulose nanofibrillaire selon l'une quelconque des revendications 1 à 5 avec un ligand ayant un groupe alcyne cyclique ou acyclique.

15. Procédé selon la revendication 14, où le procédé comprend l'étape de conjugaison d'un composé lieur ayant un groupe alcyne cyclique ou acyclique au ligand, ce qui donne ainsi le ligand ayant le groupe alcyne cyclique ou acyclique.

16. Trousse ou support solide selon la revendication 7, hydrogel de cellulose nanofibrillaire selon l'une quelconque des revendications 11 à 13, ou procédé selon la revendication 14 ou 15, où le groupe alcyne cyclique est DBCO, OCT, MOFO, DIFO, DIFO2, DIFO3, DIMAC, DIBO, BARAC, BCN, le diyne de Sondheimer, TMDIBO, S-DIBO, COMBO, ou PYRROC.

17. Utilisation de l'hydrogel de cellulose nanofibrillaire à modification azido selon l'une quelconque des revendications 1 à 5 ou de l'hydrogel de cellulose nanofibrillaire modifié par un ligand selon l'une quelconque des revendications 11 à 13 pour maintenir, transporter, isoler, cultiver, propager, repiquer, différencier ou transplanter des cellules ou des tissus.


### SEQUENCE LISTING

<110> UPM-Kymmene Corporation Glykos Finland Oy
<120> NANOFIBRILLAR CELLULOSE HYDROGEL
<130> P-EP108376M
<160> 2
<170> PatentIn version 3.5
<210> 1
   <211> 7
   <212> PRT
   <213> Artificial Sequence
<220>
   <223> An RGD adhesive peptide
<400> 1
   Gly Arg Gly Asp Ser Pro Cys
   1               5
<210> 2
   <211> 15
   <212> PRT
   <213> Artificial Sequence
<220>
   <223> Oligopeptide derived from vitronectin
<400> 2
   Lys Gly Gly Pro Gln Val Thr Arg Gly Asp Val Phe Thr Met Pro
   1               5                   10                  15